(19) Europäisches Patentamt
European Patent Office
Office européen des brevets

(11) **EP 3 626 170 A1**

(12) **EUROPEAN PATENT APPLICATION**
published in accordance with Art. 153(4) EPC

(43) Date of publication:
**25.03.2020  Bulletin 2020/13**

(51) Int Cl.:
**A61B 5/11** *(2006.01)*      **A61H 1/02** *(2006.01)*
**A61B 5/00** *(2006.01)*

(21) Application number: **17910429.4**

(22) Date of filing: **19.05.2017**

(86) International application number:
**PCT/JP2017/018935**

(87) International publication number:
**WO 2018/211713 (22.11.2018 Gazette 2018/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Fujitsu Limited**
**Kawasaki-shi, Kanagawa 211-8588 (JP)**

(72) Inventors:
• **HOTTA, Shinji**
**Kawasaki-shi**
**Kanagawa 211-8588 (JP)**
• **INOMATA, Akihiro**
**Kawasaki-shi**
**Kanagawa 211-8588 (JP)**

(74) Representative: **Haseltine Lake Kempner LLP**
**Lincoln House, 5th Floor**
**300 High Holborn**
**London WC1V 7JH (GB)**

(54) **INFORMATION PROCESSING DEVICE, INFORMATION PROCESSING SYSTEM, AND INFORMATION PROCESSING METHOD**

(57)    An information processing apparatus (100) acquires measurement information (101, 102) regarding the movement of each region of a plurality of regions of a patient. Based on the acquired measurement information, the information processing apparatus (100) detects an action group that includes an action of each region in which a feature corresponding to a part of a walking motion action of the patient appears in a target period. The information processing apparatus (100) calculates, in the detected action group, an evaluation value that indicates the degree of appearance of each relationship of a plurality of relationships produced between actions when a walking motion is performed by the patient. The information processing apparatus (100) determines, based on the evaluation value calculated for each relationship, whether or not the target period is a walking period in which a walking motion was performed by the patient.

FIG. 1

**Description**

TECHNICAL FIELD

The present invention relates to an information processing apparatus, an information processing system, and an information processing method.

BACKGROUND ART

Conventionally, in a medical field, it is required to grasp a condition of a patient in detail and precisely in order to perform an appropriate medical treatment for the patient. For example, the way of walking of a patient tends to be influenced by the degree of physical disorder due to a disease, the degree of recovery of a disease or injury, and the like, and a condition of the patient can be grasped from the way of walking of the patient. Specifically, for example, it is required to precisely grasp whether or not the patient is walking in an unnatural manner such as dragging his/her leg.

Then, in order to make it possible to precisely grasp the way of walking of the patient, an effective approach is to mount a sensor to the patient and quantify the way of walking of the patient based on data obtained from the sensor. In this quantification, it is preferable to first specify the walking period in which a walking motion was performed by the patient, and it is preferable to calculate a predetermined feature quantity in the specified walking period as an objective index used when grasping the way of walking of the patient. The predetermined feature quantity is, for example, the length of the walking period, and the number of steps, the average value of the stride, the average value of the moving speed, and the like of the patient during the walking period. An object of the present invention is to find a walking period.

As a related prior art, a conceivable case is that, using the fact that, in the walking period in which a walking motion was performed by the patient, a characteristic waveform pattern corresponding to the walking motion tends to appear in sensor data obtained from a sensor device mounted to the patient, a particular walking period is specified. For example, in Non-Patent Document 1, after extracting waveform feature quantities such as periodicity and amplitude of acceleration using accelerations obtained from sensors mounted to the waist, shins, and thighs, a model learned in advance is applied to the observed feature quantities, such that a period in which motions including walking have occurred is found. There is also a technique of successively learning from observed data without presetting a threshold or a model for finding the walking period. For example, in Non-Patent Document 2, a threshold for finding a step action equivalent to one step of walking is applied to a period provisionally determined to be likely to be walking, according to a statistical value of the observed angular velocity amplitude, and the step action is found using this threshold; then, a section where the step motion appears frequently is regarded as the walking period.

CITATION LIST

PATENT DOCUMENT

Patent Document 1: Japanese Laid-open Patent Publication No. 2016-106948
Patent Document 2: International Publication Pamphlet No. WO 2014/091583
Patent Document 3: Japanese National Publication of International Patent Application No. 2014-511189

NON-PATENT DOCUMENT

Non-Patent Document 1: Bao, "Activity Recognition from User-Annotated Acceleration Data", 2004
Non-Patent Document 2: Fraccalo, "Real-world Gyroscope-based Gait Event Detection and Gait Feature Extraction", 2014

SUMMARY OF INVENTION

TECHNICAL PROBLEM

However, when the way of walking changes depending on the patient, or the place where the patient walks, the surrounding environment when the patient walks, the condition of the patient, and the like, it tends to be difficult to specify the walking period. For example, when a patient is walking with his/her leg dragging, a waveform pattern that matches a prepared characteristic waveform pattern corresponding to the walking motion does not appear in sensor data obtained from a sensor device mounted to the patient in some cases. In addition, for example, when a patient who can usually walk in a healthy manner temporarily changes the way of walking due to a headache or the like, there is a risk that this temporary walking cannot be detected when, for example, Non-Patent Document 2 is applied.

As described above, in the prior art, it is sometimes difficult to specify the walking period in which a walking motion was performed by a patient. For example, if the way of walking changes depending on the place where the patient walks, the surrounding environment when the patient walks, the condition of the patient, and the like, it becomes difficult to specify the walking period.

In one aspect, it is an object of the present invention to provide an information processing apparatus, an information processing system, and an information processing method capable of improving accuracy of specifying a walking period.

SOLUTION TO PROBLEM

According to one form of embodiment, proposed are an information processing apparatus, an information processing system, and an information processing method that acquire measurement information regarding a movement of each region of a plurality of regions of a measurement target; based on the acquired measurement information, detect an action group that includes an action of the each region in which a feature corresponding to a part of a walking motion action of the measurement target appears in a target period; in the detected action group, calculate an evaluation value that indicates a degree of appearance of each relationship of a plurality of relationships produced between actions when a walking motion is performed by the measurement target; and based on the evaluation value calculated for the each relationship, determine whether or not the target period is a walking period in which the walking motion was performed by the measurement target.

ADVANTAGEOUS EFFECTS OF INVENTION

One form of the present invention has the effect of improving the accuracy of specifying the walking period.

BRIEF DESCRIPTION OF DRAWINGS

FIG. 1 is an explanatory diagram illustrating an example of an information processing method according to an embodiment.
FIG. 2 is an explanatory diagram illustrating an example of an information processing system 200.
FIG. 3 is a block diagram illustrating an exemplary hardware configuration of an information processing apparatus 100.
FIG. 4 is a block diagram illustrating an exemplary hardware configuration of a measuring instrument 201.
FIG. 5 is a block diagram illustrating an exemplary functional configuration of the information processing apparatus 100.
FIG. 6 is an explanatory diagram illustrating an example of an operation flow of the information processing apparatus 100.
FIG. 7 is an explanatory diagram illustrating an example of a first relationship between actions.
FIG. 8 is an explanatory diagram illustrating an example of a second relationship between actions.
FIG. 9 is an explanatory diagram illustrating an example of a third relationship between actions.
FIG. 10 is an explanatory diagram illustrating an example of a fourth relationship between actions.
FIG. 11 is an explanatory diagram illustrating an example of a fifth relationship between actions.
FIG. 12 is an explanatory diagram illustrating an example of a sixth relationship between actions.
FIG. 13 is an explanatory diagram illustrating an example of a seventh relationship between actions.
FIG. 14 is an explanatory diagram illustrating an example of an eighth relationship between actions.
FIG. 15 is an explanatory diagram illustrating an example of a ninth relationship between actions.
FIG. 16 is an explanatory diagram illustrating an example of a tenth relationship between actions.
FIG. 17 is an explanatory diagram illustrating an example in which the information processing apparatus 100 determines whether or not a target period is a walking period.
FIG. 18 is an explanatory diagram (Part 1) illustrating a first specific example in which the information processing apparatus 100 specifies a walking period.
FIG. 19 is an explanatory diagram (Part 2) illustrating the first specific example in which the information processing apparatus 100 specifies a walking period.

FIG. 20 is an explanatory diagram (Part 3) illustrating the first specific example in which the information processing apparatus 100 specifies a walking period.

FIG. 21 is an explanatory diagram (Part 4) illustrating the first specific example in which the information processing apparatus 100 specifies a walking period.

FIG. 22A is an explanatory diagram (Part 5) illustrating the first specific example in which the information processing apparatus 100 specifies a walking period.

FIG. 22B is an explanatory diagram (Part 6) illustrating the first specific example in which the information processing apparatus 100 specifies a walking period.

FIG. 23 is an explanatory diagram (Part 7) illustrating the first specific example in which the information processing apparatus 100 specifies a walking period.

FIG. 24 is an explanatory diagram (Part 8) illustrating the first specific example in which the information processing apparatus 100 specifies a walking period.

FIG. 25 is an explanatory diagram (Part 9) illustrating the first specific example in which the information processing apparatus 100 specifies a walking period.

FIG. 26 is an explanatory diagram (Part 10) illustrating the first specific example in which the information processing apparatus 100 specifies a walking period.

FIG. 27 is an explanatory diagram (Part 1) illustrating a second specific example in which the information processing apparatus 100 specifies a walking period.

FIG. 28 is an explanatory diagram (Part 2) illustrating the second specific example in which the information processing apparatus 100 specifies a walking period.

FIG. 29 is an explanatory diagram (Part 3) illustrating the second specific example in which the information processing apparatus 100 specifies a walking period.

FIG. 30 is an explanatory diagram (Part 4) illustrating the second specific example in which the information processing apparatus 100 specifies a walking period.

FIG. 31 is an explanatory diagram (Part 5) illustrating the second specific example in which the information processing apparatus 100 specifies a walking period.

FIG. 32 is an explanatory diagram (Part 6) illustrating the second specific example in which the information processing apparatus 100 specifies a walking period.

FIG. 33 is an explanatory diagram (Part 7) illustrating the second specific example in which the information processing apparatus 100 specifies a walking period.

FIG. 34 is an explanatory diagram illustrating an example in which the information processing apparatus 100 outputs a predetermined feature quantity.

FIG. 35 is an explanatory diagram (Part 1) illustrating another example of the operation of applying a plurality of band pass filters.

FIG. 36 is an explanatory diagram (Part 2) illustrating another example of the operation of applying a plurality of band pass filters.

FIG. 37 is an explanatory diagram illustrating another example of the operation of setting a plurality of time length upper limit candidates for a time length upper limit of a step action.

FIG. 38 is a flowchart illustrating an example of an overall processing procedure.

FIG. 39 is a flowchart illustrating an example of a walking period specifying procedure.

FIG. 40 is a flowchart illustrating an example of an evaluation value calculation processing procedure.

FIG. 41 is a flowchart illustrating an example of a likelihood calculation processing procedure.

DESCRIPTION OF EMBODIMENTS

Hereinafter, embodiments of an information processing apparatus, an information processing system, and an information processing method according to the present invention will be described in detail with reference to the drawings.

(One Example of Information Processing Method According to Embodiment)

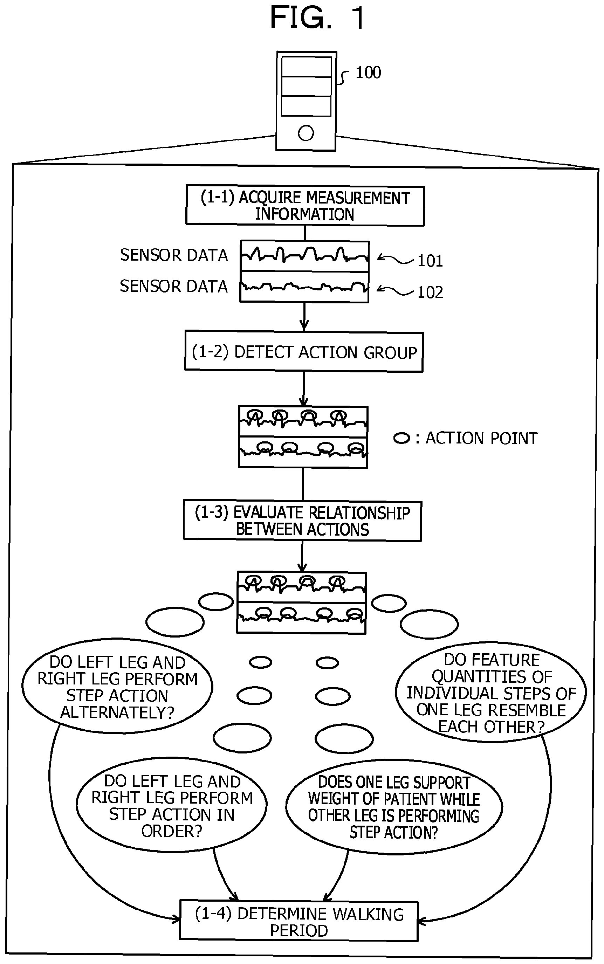

FIG. 1 is an explanatory diagram illustrating an example of an information processing method according to an embodiment. An information processing apparatus 100 is a computer that specifies a walking period in which walking is performed by a measurement target. The measurement target is a living body or an artificial thing. The living body may be human or non-human animals. For example, the living body is a subject to be examined by a medical institution. Specifically, the living body is a patient who receives diagnosis, medical care, follow-up, health management, or the like by a medical person such as a medical doctor. The artificial thing is, for example, a biped robot.

Specifically, the living body may be a patient who receives rehabilitation instruction or the like from a medical person such as a medical doctor. Alternatively, the living body may specifically be a trainee who is given a walking instruction by a sports instructor. The living body may be, for example, an individual who performs his/her own health management. The living body may be an animal. In the following description, mainly, a case where the measurement target is a "patient" will be described.

The present embodiment will describe an information processing method that can improve the accuracy of specifying the walking period, by evaluating the degree of appearance of each relationship of a plurality of relationships produced between actions of regions of the patient when a walking motion is performed by the patient.

(1-1) The information processing apparatus 100 acquires measurement information regarding the movement of each region of a plurality of regions of a patient. The plurality of regions of the patient is, for example, two regions of the patient's left leg and right leg. The plurality of regions of the patient is specifically two regions of the lower leg of the left leg and the lower leg of the right leg of the patient. The measurement information regarding the movement of a region of the patient is, for example, time-series data in which angular velocities measured by an angular velocity sensor of a measuring instrument mounted to the region are arranged in time series. The time-series data is, for example, sensor data.

The information processing apparatus 100 receives, for example, sensor data 101 containing the angular velocity of the patient's lower leg of the left leg from a measuring instrument mounted to the patient's lower leg of the left leg, and receives sensor data 102 containing the angular velocity of the patient's lower leg of the right leg from a measuring instrument mounted to the patient's lower leg of the right leg. With this configuration, the information processing apparatus 100 can use the sensor data to specify the walking period of the patient.

(1-2) Based on the acquired measurement information, the information processing apparatus 100 detects an action group that includes an action of each region in which a feature corresponding to a part of a walking motion action of the patient appears in a target period. A part of the walking motion action is, for example, one step of walking motion. A part of the walking motion action may be, for example, the resting state of the support leg during the walking motion, or the two-leg tremor when both feet are on the ground during the walking motion.

The feature corresponding to a part of the walking motion action is, for example, a feature corresponding to one step of the walking motion. The feature corresponding to one step of the walking motion is, for example, that the angular velocity exceeds a threshold and reaches a local maximum value. The action of the region is the action of the patient's left or right leg moving ahead by one step. The feature corresponding to a part of the walking motion action is, for example, a feature corresponding to the two-leg tremor when both feet are on the ground during the walking motion. The feature corresponding to the two-leg tremor is, for example, that the angular velocity is equal to or less than the threshold and the vibration is equal to or more than a threshold.

In the following description, an action of moving ahead by one step is sometimes referred to as a "step action". The step action is an action from a time point when a leg leaves the ground to a time point when the leg lands after the leg swings. A time point when the leg leaves the ground is the start time of the step action. A time point when the leg lands is the end time of the step action. The action group is, for example, a step action group.

The information processing apparatus 100 detects, for example, in the sensor data 101 containing the angular velocity of the patient's lower leg of the left leg, that the angular velocity has exceeded the threshold and reached a local maximum value in the target period, and detects that a step action of the left leg has been performed. Upon detecting that a step action of the left leg has been performed, the information processing apparatus 100 stores an action point at which the detected step action was performed.

The information processing apparatus 100 detects, for example, in the sensor data 102 containing the angular velocity of the patient's lower leg of the right leg, that the angular velocity has exceeded the threshold and reached a local maximum value in the target period, and detects that a step action of the right leg has been performed. Upon detecting that a step action of the right leg has been performed, the information processing apparatus 100 stores an action point at which the detected step action was performed. With this configuration, the information processing apparatus 100 can detect a step action group that includes step actions of the respective legs, and can use the detected step action group to specify the walking period of the patient.

(1-3) The information processing apparatus 100 calculates, in the detected action group, an evaluation value that indicates the degree of appearance of each relationship of a plurality of relationships produced between actions when a walking motion is performed by the patient. The plurality of relationships includes, for example, a first relationship in which the step action of the left leg alternates with the step action of the right leg. The plurality of relationships includes, for example, a second relationship in which the step action of one leg starts after the step action of the other leg ends.

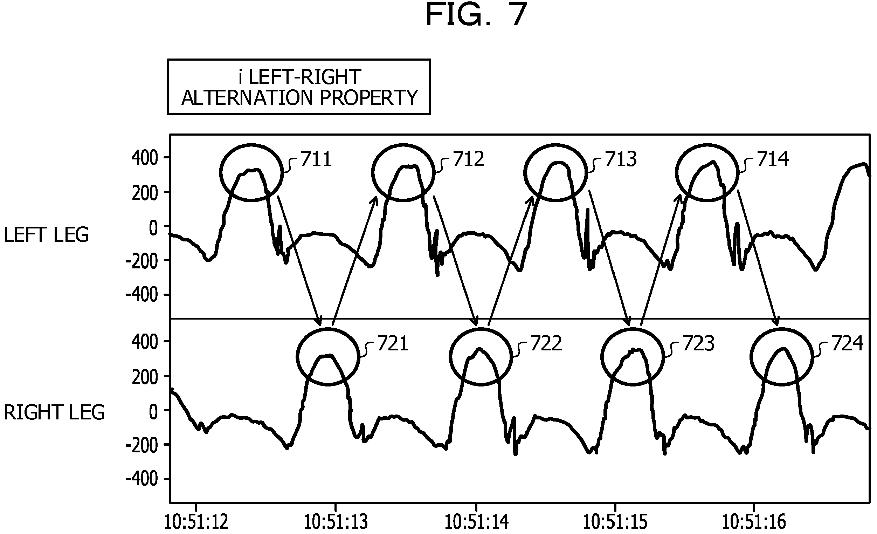

The plurality of relationships includes, for example, a third relationship in which one leg serves as a support leg and the amount of action of the one leg is smaller than a predetermined minute value during the step action of the other leg. The amount of action is, for example, an angular velocity. The plurality of relationships includes, for example, a fourth relationship in which the feature quantities of different step actions of one leg resemble each other. Specific examples of the plurality of relationships will be described later with reference to FIGs. 7 to 16. With these relationships, the information processing apparatus 100 can evaluate the likeliness as a walking period in which a walking motion was performed by the patient, from a variety of viewpoints.

(1-4) The information processing apparatus 100 determines, based on the evaluation value calculated for each relationship, whether or not the target period is a walking period in which a walking motion was performed by the patient. For example, the information processing apparatus 100 determines that the target period is a walking period in which a walking motion was performed by the patient, when the evaluation value calculated for each relationship exceeds a threshold serving as determination criteria.

With these evaluation values, the information processing apparatus 100 evaluates the likeliness of the walking period from a variety of viewpoints and accordingly can easily specify the walking period with higher accuracy. For example, since the evaluation values calculated for the second relationship and the third relationship are also used even when the first relationship appears while the patient performs a non-walking motion resembling walking, such as a motion of riding a bicycle, the information processing apparatus 100 can determine that a period in which the above non-walking motion was performed is not a walking period, and can improve the accuracy of specifying the walking period. Meanwhile, since all of the first to tenth relationships have higher evaluation values even when, for example, the patient walks with his/her leg dragging or walks at an extremely low speed, the information processing apparatus 100 can determine that a period in which the above-mentioned motions were performed is a walking period, and can improve the accuracy of specifying the walking period.

Furthermore, the information processing apparatus 100 can calculate a predetermined feature quantity in the specified walking period and output the calculated feature quantity, thereby being able to give a medical person such as a medical doctor an objective index used when grasping the way of walking of the patient. The information processing apparatus 100 can output, for example, the length of the specified walking period, and the number of steps, the average value of the stride, the average value of the moving speed, and the like of the patient during the specified walking period. Therefore, the information processing apparatus 100 can make it easier for a medical person such as a medical doctor to grasp the way of walking of the patient, and can allow the medical person to efficiently perform diagnosis, medical care, follow-up, health management, or the like for the patient.

In addition, the information processing apparatus 100 can similarly output, to a medical person such as a medical doctor, for example, the length of daily walking period of the patient, and the number of steps, the average value of the stride, the average value of the moving speed, and the like of the patient during the walking period including outside the hospital. Therefore, the information processing apparatus 100 can make it easier for a medical person such as a medical doctor to grasp the way of daily walking of the patient, and to grasp the daily motion amount of the patient, and can allow the medical person to perform diagnosis, medical care, follow-up, health management, or the like for the patient in detail and precisely.

Here, the case where the measuring instruments are mounted to the left leg and the right leg of the patient has been described, but the present invention is not limited to this. For example, the measuring instruments may be mounted to one leg and the waist of the patient. Alternatively, for example, the measuring instruments may be mounted to the left leg, the right leg, and the waist of the patient. In this case, the information processing apparatus 100 can detect a body trunk advancing action of the waist corresponding to the step action of each leg in order to specify the walking period of the patient. The body trunk advancing action of the waist is an action of moving the body trunk before and after the step action. In this case, the action group may include the body trunk advancing action of the waist. In addition, a measuring instrument mounted to the arm or head of the patient may be adopted.

Here, the way of walking of the patient is different depending on the place where the patient walks, the surrounding environment when the patient walks, the condition of the patient, and the like and specifically, there are cases where the cycle of step action of each leg of the patient is different or the sensor data contains a noise signal when the leg is dragged on the ground. In addition, the cycle of step action of each leg of the patient may be different for each patient. For this reason, when detecting the action group in (1-2), the information processing apparatus 100 applies each band pass filter of a plurality of band pass filters to the measurement information regarding the movement of a region of the patient, and consequently can further improve the accuracy of specifying the walking period.

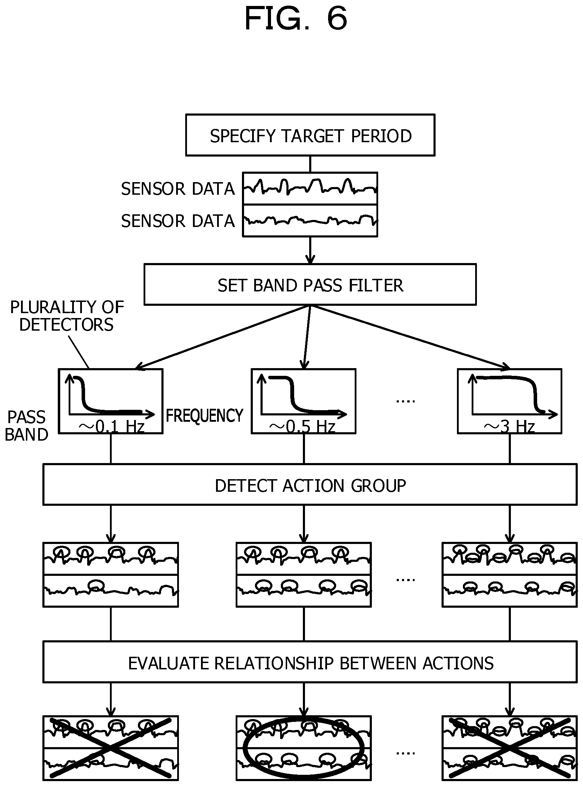

For example, the information processing apparatus 100 detects the step action group for a case where each band pass filter is applied to the measurement information regarding the movement of a region of the patient. Then, the information processing apparatus 100 calculates, for each of the detected step action groups, an evaluation value that indicates the degree of appearance of each relationship, and determines whether or not the target period is a walking period. A specific example in which the information processing apparatus 100 uses a plurality of band pass filters will be described later with reference to FIGs. 6 to 26.

In addition, the way of walking of the patient differs depending on the place where the patient walks, the surrounding environment when the patient walks, the condition of the patient, and the like and specifically, there are cases where the time length of step action of each leg of the patient is different. In addition, the time length of step action of each leg of the patient may be different for each patient. For this reason, the information processing apparatus 100 sets a time length upper limit of the step action to each candidate of a plurality of time length upper limit candidates, and consequently can further improve the accuracy of specifying the walking period.

For example, the information processing apparatus 100 calculates an evaluation value that indicates the degree of appearance of each relationship, for a case where the time length upper limit of the step action is set to each candidate of a plurality of time length upper limit candidates, and determines whether or not the target period is a walking period. A specific example in which the information processing apparatus 100 uses a plurality of time length upper limit candidates will be described later with reference to FIGs. 6 to 26.

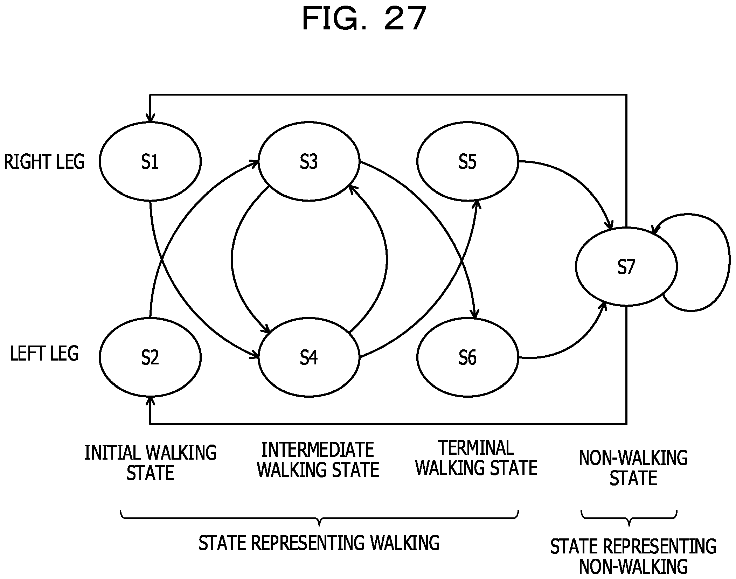

In addition, it is conceivable that the target period includes both of a walking period in which a walking motion was performed by the patient and a non-walking period in which no walking motion was performed by the patient. Therefore, the information processing apparatus 100 may determine which partial period of the target period is a walking period, based on the evaluation value calculated for each relationship. A specific example in which the information processing apparatus 100 determines which partial period of the target period is a walking period will be described later with reference to FIGs. 27 to 33.

(One Example of Information Processing System 200)

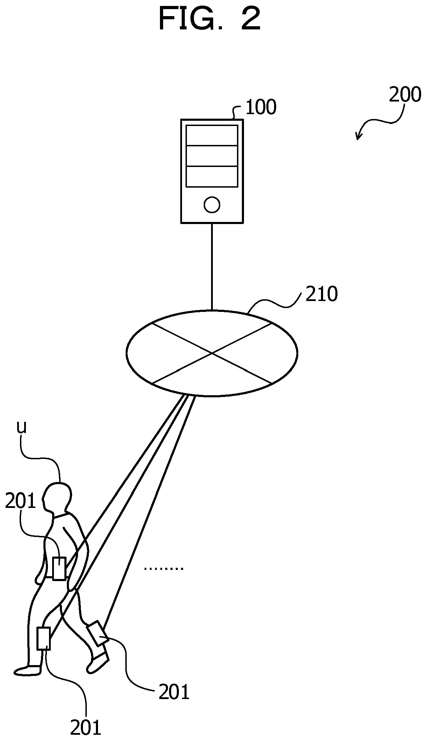

Next, an example of an information processing system 200 to which the information processing apparatus 100 illustrated in FIG. 1 is applied will be described with reference to FIG. 2.

FIG. 2 is an explanatory diagram illustrating an example of the information processing system 200. In FIG. 2, the information processing system 200 includes the information processing apparatus 100 and one or more measuring instruments 201. In the information processing system 200, the information processing apparatus 100 and the measuring instrument 201 are connected via a wired or wireless network 210. Examples of the network 210 include a local area network (LAN), a wide area network (WAN), and the Internet.

The information processing apparatus 100 is a computer that acquires, from one or more measuring instruments 201, measurement information regarding the movement of each region of a plurality of regions of a patient to be a measurement target u, and specifies a walking period in which a walking motion was performed by the patient. Examples of the information processing apparatus 100 include a server, a personal computer (PC), a notebook PC, a tablet terminal, a smartphone, and a wearable terminal.

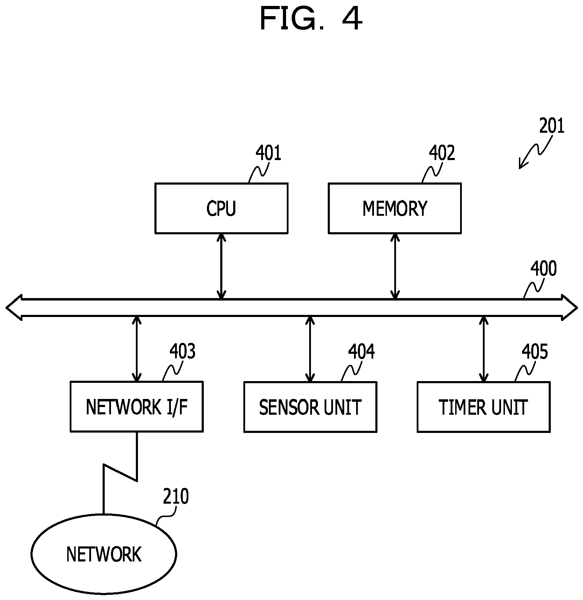

The measuring instrument 201 is a computer mounted to a patient to be the measurement target u. The measuring instrument 201 generates measurement information and transmits the measurement information to the information processing apparatus 100. The measuring instrument 201 includes, for example, a sensor unit illustrated in FIG. 4, generates time-series data in which a measurement value of the sensor unit and a measurement time at which the measurement value of the sensor unit is obtained are associated with each other, and transmits the time-series data to the information processing apparatus 100 as measurement information. The measuring instrument 201 is a sensor device, for example. The sensor device is specifically a device called a motion sensor. The measuring instrument 201 may be a smartphone or a wearable terminal, for example.

Here, the case where the measuring instrument 201 creates time-series data has been described, but the present invention is not limited to this. For example, the information processing apparatus 100 may sequentially receive, from the measuring instrument 201, correspondence information in which a measurement value of the sensor unit of the measuring instrument 201 and a measurement time at which the measurement value of the sensor unit is obtained are associated with each other, and create time-series data in which the correspondence information is compiled. Here, the case where the information processing apparatus 100 and the measuring instrument 201 are different devices has been described, but the present invention is not limited to this. For example, the information processing apparatus 100 may be integrated with the measuring instrument 201.

Here, the case where the measuring instrument 201 generates measurement information regarding the movement of the region of the patient on which the measuring instrument 201 is mounted has been described, but the present invention is not limited to this. For example, even if the measuring instrument 201 is mounted to the patient's waist, the measuring instrument 201 can acquire accelerations corresponding to the movement of the patient's left leg and right leg by analyzing the measurement value of an acceleration sensor, and may generate measurement information regarding the movement of the patient's left leg and right leg. In this case, the information processing system 200 can be implemented even if only one measuring instrument 201 is used for each patient.

The information processing system 200 may further include a display device. In this case, as a result of specifying the walking period, the information processing apparatus 100 displays a predetermined feature quantity related to the walking period, and the like via the display device. Examples of the display device include a PC, a notebook PC, a tablet terminal, or a smartphone.

For example, the information processing system 200 is applied to a medical system of a medical institution, or is applied to implement a watching service for grasping a condition of a patient, or is applied to implement a service for a personal health record (PHR).

(Exemplary Hardware Configuration of Information Processing Apparatus 100)

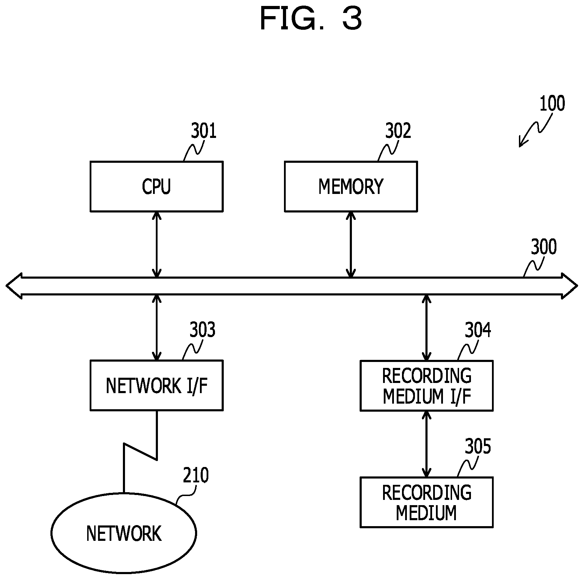

Next, an exemplary hardware configuration of the information processing apparatus 100 included in the information processing system 200 illustrated in FIG. 2 will be described with reference to FIG. 3.

FIG. 3 is a block diagram illustrating an exemplary hardware configuration of the information processing apparatus 100. In FIG. 3, the information processing apparatus 100 includes a central processing unit (CPU) 301, a memory 302, a network interface (I/F) 303, a recording medium I/F 304, and a recording medium 305. In addition, each of components is interconnected by a bus 300.

Here, the CPU 301 performs overall control of the information processing apparatus 100. The memory 302 includes a read only memory (ROM), a random access memory (RAM), and a flash ROM, for example. Specifically, for example, the flash ROM or the ROM stores various programs, while the RAM is used as a work area for the CPU 301. A program stored in the memory 302 is loaded into the CPU 301 to cause the CPU 301 to execute coded processing.

The network I/F 303 is connected to the network 210 through a communication line, and is connected to another computer through the network 210. In addition, the network I/F 303 manages an interface between the network 210 and an inside, and controls input-output of data from another computer. Examples of the network I/F 303 include a modem or a LAN adapter.

The recording medium I/F 304 controls read and write of data toward the recording medium 305 under the control of the CPU 301. Examples of the recording medium I/F 304 include a disk drive, a solid state drive (SSD), or a universal serial bus (USB) port. The recording medium 305 is a nonvolatile memory that stores data written under the control of the recording medium I/F 304. Examples of the recording medium 305 include a disk, a semiconductor memory, or a USB memory. The recording medium 305 may be removably installed on the information processing apparatus 100.

The information processing apparatus 100 may further include a keyboard, a mouse, a display, a printer, a speaker, or a touch panel, for example, in addition to the components described above. In addition, the information processing apparatus 100 may omit the recording medium I/F 304 or the recording medium 305.

(Exemplary Hardware Configuration of Measuring Instrument 201)

Next, an exemplary hardware configuration of the measuring instrument 201 included in the information processing system 200 illustrated in FIG. 2 will be described with reference to FIG. 4.

FIG. 4 is a block diagram illustrating an exemplary hardware configuration of the measuring instrument 201. In FIG. 4, the measuring instrument 201 includes a CPU 401, a memory 402, a network I/F 403, a sensor unit 404, and a timer unit 405. In addition, each of components is interconnected by a bus 400.

Here, the CPU 401 performs overall control of the measuring instrument 201. The memory 402 includes, for example, a ROM, a RAM, and a flash ROM. Specifically, for example, the flash ROM or the ROM stores various programs, while the RAM is used as a work area for the CPU 401. A program stored in the memory 402 is loaded into the CPU 401 to cause the CPU 401 to execute coded processing.

The network I/F 403 is connected to the network 210 through a communication line, and is connected to another computer through the network 210. In addition, the network I/F 403 manages an interface between the network 210 and an inside, and controls input-output of data from another computer. Examples of the network I/F 403 include a communication circuit corresponding to Wi-Fi (registered trademark), or a communication circuit corresponding to Bluetooth (registered trademark). The network I/F 403 may be, for example, a communication circuit corresponding to the 3rd Generation (3G).

The sensor unit 404 measures a condition of the measuring instrument 201. The sensor unit 404 measures, for example, at least one of a position, a movement, and an orientation of the measuring instrument 201. Specifically, the sensor unit 404 includes at least one of an acceleration sensor, an angular velocity sensor, a terrestrial magnetism sensor, an optical sensor, a vibration sensor, and the like. In addition, the sensor unit 404 may include a global positioning systems (GPS) receiver, and may find GPS coordinates of the measuring instrument 201. The timer unit 405 measures a current time.

The measuring instrument 201 may further include a keyboard, a mouse, a display, a printer, a speaker, or a touch panel, for example, in addition to the components described above. In addition, the measuring instrument 201 may further include a recording medium I/F or a recording medium, in addition to the components described above. The recording medium may be removably installed on the measuring instrument 201.

(Exemplary Functional Configuration of Information Processing Apparatus 100)

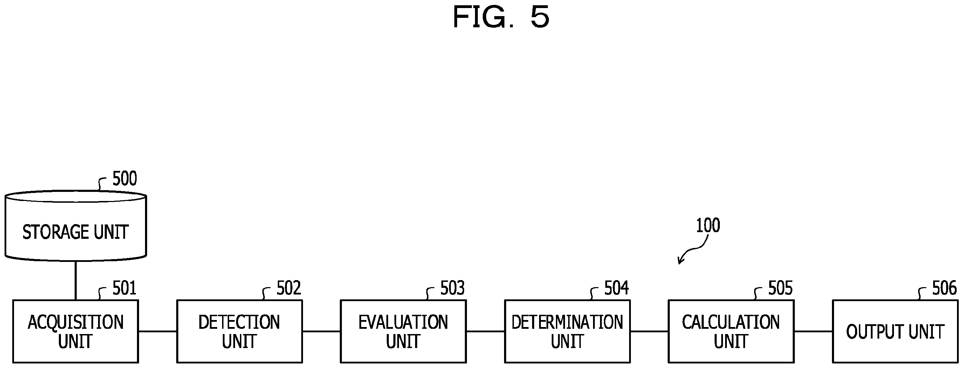

Next, an exemplary functional configuration of the information processing apparatus 100 will be described with reference to FIG. 5.

FIG. 5 is a block diagram illustrating an exemplary functional configuration of the information processing apparatus 100. The information processing apparatus 100 includes a storage unit 500, an acquisition unit 501, a detection unit 502, an evaluation unit 503, a determination unit 504, a calculation unit 505, and an output unit 506.

For example, the storage unit 500 is implemented by a storage area such as the memory 302 or the recording medium 305 illustrated in FIG. 3. The acquisition unit 501 to the output unit 506 have functions as a control unit. Specifically, for example, the acquisition unit 501 to the output unit 506 implement functions thereof by causing the CPU 301 to execute a program stored in a storage area such as the memory 302 or the recording medium 305, or by the network I/F 303, illustrated in FIG. 3. A processing result of individual functional units is stored in a storage area such as the memory 302 or the recording medium 305, illustrated in FIG. 3, for example.

The storage unit 500 stores measurement information regarding the movement of each region of a plurality of regions of a measurement target. The measurement target is, for example, a patient. The plurality of regions is, for example, the left leg and the right leg. The plurality of regions may include the waist. The storage unit 500 stores, for example, sensor data in which a measurement value of the sensor unit of the measuring instrument 201 and a measurement time at which the measurement value is obtained are associated with each other and arranged in time series.

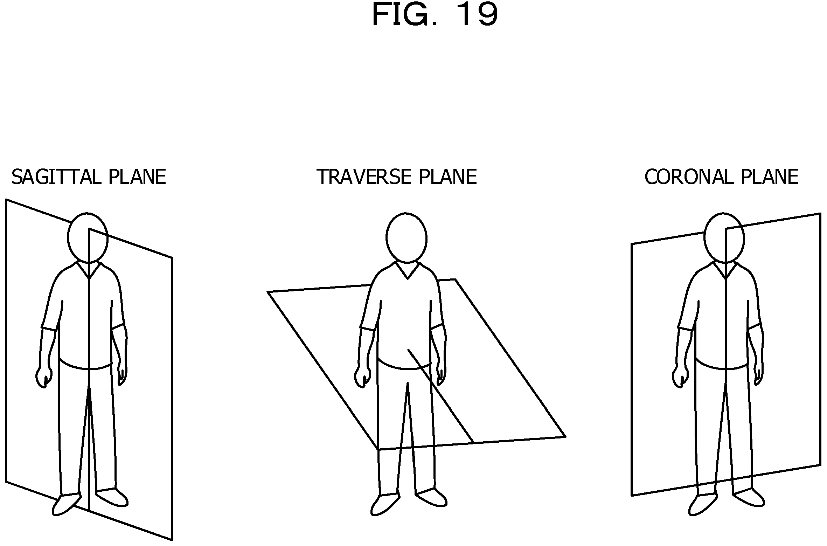

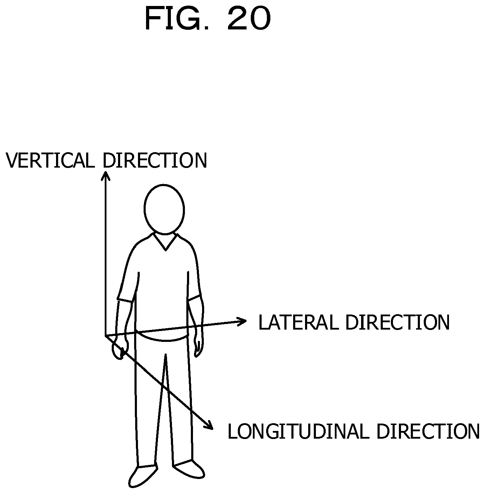

The measurement value includes, for example, at least one of the angular velocity on a sagittal plane, the angular velocity on a traverse plane, and the angular velocity on a coronal plane. The measurement value may include, for example, at least one of acceleration in a vertical direction, acceleration in a lateral direction, and acceleration in a longitudinal direction. In the following description, vertical acceleration, lateral acceleration, and longitudinal acceleration are sometimes referred to as "vertical acceleration", "lateral acceleration", and "longitudinal acceleration", respectively. The measurement value may include, for example, the magnitude and position of vibration. With this configuration, the storage unit 500 can allow the detection unit 502 to refer to sensor data. Note that the definitions of the sagittal plane, the traverse plane, and the coronal plane are as illustrated in FIG. 19, and the definitions of the vertical direction, the lateral direction, and the longitudinal direction are as illustrated in FIG. 20.

The storage unit 500 stores information specifying each band pass filter of a plurality of band pass filters. The storage unit 500 stores a first parameter indicating a frequency component extracted by each band pass filter of the plurality of band pass filters. With this configuration, the storage unit 500 can allow the detection unit 502 to refer to the first parameter, and can make each band pass filter of the plurality of band pass filters available.

The storage unit 500 stores each candidate of a plurality of time length upper limit candidates for the time length upper limit of the step action. The storage unit 500 stores a second parameter indicating each candidate of the plurality of time length upper limit candidates. The second parameter indicates the time length upper limit of the step action. With this configuration, the storage unit 500 can allow the detection unit 502 to refer to the second parameter, and can allow each candidate of the plurality of time length upper limit candidates to be set as the time length upper limit of the step action.

The storage unit 500 stores a parameter group of a calculation formula for calculating the evaluation value for each relationship of the plurality of relationships. Each of the plurality of relationships is a relationship with a high possibility of being produced between actions when a walking motion is performed by the patient. The plurality of relationships includes, for example, first to tenth relationships described later with reference to FIGs. 7 to 16. The plurality of relationships may include at least two of the first to tenth relationships described later with reference to FIGs. 7 to 16. With this configuration, the storage unit 500 can allow the evaluation unit 503 to refer to the parameter group of the calculation formula.

The acquisition unit 501 acquires measurement information regarding the movement of each region of a plurality of regions of a patient. The acquisition unit 501 receives, from the measuring instrument 201, for example, sensor data in which a measurement value of the sensor unit of the measuring instrument 201 and a measurement time at which the measurement value is obtained are associated with each other and arranged in time series, and stores the received sensor data in the storage unit 500. For example, the acquisition unit 501 may sequentially receive, from the measuring instrument 201, correspondence information in which a measurement value of the sensor unit of the measuring instrument 201 and a measurement time at which the measurement value is obtained are associated with each other, create sensor data in which the correspondence information is compiled, and store the sensor data in the storage unit 500. With this configuration, the acquisition unit 501 can allow the detection unit 502, the evaluation unit 503, and the like to refer to sensor data for each region of the patient.

The detection unit 502 sets a target period to be determined as to whether or not the target period is a walking period. The detection unit 502 sets a predetermined period as a target period. The detection unit 502 may set a period based on the user's operation input as a target period. The detection unit 502 may specify an action period in which the patient is not in a stationary state, based on the acquired measurement information, and set the specified action period as a target period.

Based on the acquired measurement information, the detection unit 502 detects an action group that includes an action of each region in which a feature corresponding to a part of a walking motion action of the patient appears in the target period. A part of the walking motion action is, for example, one step of walking motion. A part of the walking motion action may be, for example, the resting state of the support leg during the walking motion, or the two-leg tremor when both feet are on the ground during the walking motion. The feature corresponding to a part of the walking motion action is, for example, a feature corresponding to one step of the walking motion. The feature corresponding to one step of the walking motion is, for example, that the angular velocity of each leg exceeds a threshold and reaches a local maximum value.

The feature corresponding to a part of the walking motion action may be, for example, a feature corresponding to the two-leg tremor when both feet are on the ground during the walking motion. The feature corresponding to the two-leg tremor is, for example, that the angular velocity is equal to or less than the threshold and the vibration is equal to or more than a threshold. The action is, for example, a step action. The step action of the left leg or the right leg is an action of the left leg or the right leg moving ahead by one step. The detection unit 502 stores the detected step action using the serial number allocated to the detected step action and the action point at which the detected step action was performed.

The detection unit 502 detects that the angular velocity of the left leg has exceeded the threshold and reached the local maximum value in the sensor data for the left leg, and detects the fact that the angular velocity of the left leg has exceeded the threshold and reached the local maximum value, as a step action of the left leg. Similarly, the detection unit 502 detects the step action of the right leg. Then, the detection unit 502 detects a step action group that includes the step action of the left leg and the step action of the right leg, as an action group. With this procedure, the detection unit 502 can allow the evaluation unit 503 to refer to the action group used when determining the walking motion.

The detection unit 502 may further detect the body trunk advancing actions of the waist before and after the step action based on the sensor data for the waist. The body trunk advancing action of the waist is an action of moving the body trunk in response to the left leg or the right leg moving ahead by one step, and occurs before and after the step action. When using the body trunk advancing action of the waist to calculate the evaluation value, the detection unit 502 detects the step action group and then detects the body trunk advancing action of the waist corresponding to each step action in the step action group, thereby detecting an action group that includes the step action group and a body trunk advancing action group. With this procedure, the detection unit 502 can allow the evaluation unit 503 to refer to the action group used when determining the walking motion.

For example, for a case where each band pass filter of a plurality of band pass filters is applied to sensor data, the detection unit 502 detects an action group that includes an action of each region in which a feature corresponding to a part of a walking motion action of the patient appears. For example, for a case where each band pass filter of a plurality of band pass filters is applied to sensor data, the detection unit 502 detects an action group that includes an action of each region in which a feature corresponding to one step of the walking motion of the patient appears. Specifically, the detection unit 502 applies each band pass filter of the plurality of band pass filters to sensor data, using each first parameter of a plurality of first parameters in the storage unit 500. With this procedure, the detection unit 502 can cope with a case where the way of walking is different for each patient, and the cycle of the step action of each leg is different for each patient, and a case where the sensor data contains a noise signal, and can facilitate the detection of the step action of each leg.

The evaluation unit 503 calculates, in the detected action group, an evaluation value that indicates the degree of appearance of each relationship of a plurality of relationships produced between actions when a walking motion is performed by the patient. The evaluation value may be, for example, a binary truth value that indicates whether or not a relationship has appeared. Specifically, an evaluation value of 1 indicates that the result is true, which indicates that a relationship has appeared. With this procedure, the evaluation unit 503 can acquire an index indicating the likeliness to the walking period of the target period.

When the detection unit 502 detects a plurality of action groups, the evaluation unit 503 may calculate an evaluation value that indicates the degree of appearance of each relationship for each of the action groups. With this procedure, the evaluation unit 503 can cope with the difference in the way of walking for each patient, and acquire an index indicating the likeliness to the walking period of the target period.

The evaluation unit 503 may calculate, in the action group, an evaluation value that indicates the degree of appearance of each relationship, for a case where the time length upper limit of each step action is set to each candidate of a plurality of time length upper limit candidates. For example, using each second parameter of a plurality of second parameters in the storage unit 500, the evaluation unit 503 calculates an evaluation value that indicates the degree of appearance of each relationship, for a case where the time length upper limit of the step action is set to each candidate of a plurality of time length upper limit candidates. With this procedure, the evaluation unit 503 can cope with the difference in the way of walking for each patient, and acquire an index indicating the likeliness to the walking period of the target period.

The determination unit 504 determines, based on the evaluation value calculated for each relationship, whether or not the target period is a walking period. The determination unit 504 determines that the target period is a walking period, for example, when the evaluation value calculated for each relationship is equal to or more than a threshold. With this configuration, the determination unit 504 can improve the accuracy of specifying the walking period.

For example, the determination unit 504 may determine whether or not the target period is a walking period, based on the result of logical operation using the evaluation value. Specifically, if the evaluation value has a binary truth value, the determination unit 504 determines that the target period is a walking period when the logical product is 1. With this configuration, the determination unit 504 can reduce the amount of processing required for the determination. In addition, since the range of values that can be taken by the evaluation value is extremely limited, the determination unit 504 can also make it easier to manually set an observation probability parameter of a state transition model described later.

For example, based on the evaluation value calculated for each relationship, the determination unit 504 may specify a candidate period for the walking period from the target period, calculate the likelihood as a walking period according to the length of the specified candidate period, and determine whether or not the candidate period is a walking period based on the likelihood. With this processing, the determination unit 504 can improve the accuracy of specifying the walking period even when the target period includes the walking period and the non-walking period.

For example, the determination unit 504 may determine whether or not the target period is a walking period in which a walking motion was performed by the patient, based on the result of logical operation using the evaluation value. With this configuration, the determination unit 504 can reduce the amount of processing required for the determination.

The calculation unit 505 calculates a predetermined feature quantity in a target period determined by the determination unit 504 to be a walking period. The predetermined feature quantity is a feature quantity related to the walking period, a feature quantity related to the movement of the patient during the walking period, and the like. The predetermined feature quantity is, for example, the length of the walking period, and the number of steps, the average value of the stride, the average value of the moving speed, and the like of the patient during the walking period.

The output unit 506 outputs the predetermined feature quantity calculated by the calculation unit 505. The output unit 506 may output the predetermined feature quantities such as the start time and end time of each step action during the walking period. The output is implemented as display on a display, print output to a printer, transmission to an external device by the network I/F 303, or storage to a storage area such as the memory 302 and the recording medium 305, for example.

(One Example of Operation Flow of Information Processing Apparatus 100)

Next, an example of an operation flow of the information processing apparatus 100 will be described with reference to FIGs. 6 to 17.

FIG. 6 is an explanatory diagram illustrating an example of an operation flow of the information processing apparatus 100. In FIG. 6, the information processing apparatus 100 receives, from the measuring instrument 201, sensor data in which the angular velocities and accelerations of the patient's waist are arranged in time series, sensor data in which the angular velocities and accelerations of the patient's left leg are arranged in time series, sensor data in which the angular velocities and accelerations of the patient's right leg are arranged in time series, and the like.

In the following description, sensor data in which accelerations are arranged in time series is sometimes referred to as "acceleration data", and sensor data in which angular velocities are arranged in time series is sometimes referred to as "gyro data". Then, based on the received sensor data, the information processing apparatus 100 specifies an action period in which the patient is not in a stationary state, and sets the specified action period as a target period to be determined as to whether or not the specified action period is a walking period.

For example, the information processing apparatus 100 receives, as sensor data, gyro data on the sagittal plane, gyro data on the traverse plane, and gyro data on the coronal plane. For example, the information processing apparatus 100 receives, as sensor data, acceleration data in the vertical direction, acceleration data in the lateral direction, and acceleration data in the longitudinal direction.

Next, the information processing apparatus 100 applies each band pass filter of a plurality of band pass filters to be detectors, to sensor data for the target period, using a plurality of first parameters. The first parameter specifies a frequency band to be attenuated and a frequency band to pass. Then, the information processing apparatus 100 detects a step action group that includes step actions of the waist, the left leg, and the right leg of the patient, for a case where each band pass filter is applied.

The gyro data used to apply the band pass filter and to detect the step action may be any one of gyro data on the sagittal plane, gyro data on the traverse plane, and gyro data on the coronal plane. Also, gyro data not used to detect the step action is used to calculate the evaluation value.

In addition, the gyro data used to apply the band pass filter and to detect the step action may be gyro data obtained by merging the gyro data on the sagittal plane, the gyro data on the traverse plane, and the gyro data on the coronal plane.

Next, the information processing apparatus 100 sets the time length upper limit of the step action using the second parameter for each of the detected step action groups, specifies the start time and end time of each step action, and calculates an evaluation value that indicates the degree of appearance of each relationship of a plurality of relationships between actions. In this case, the plurality of relationships includes the first to sixth relationships described later with reference to FIGs. 7 to 12. Then, the information processing apparatus 100 determines, based on the evaluation value, whether or not the target period is a walking period.

In addition, the information processing apparatus 100 may further detect, based on the sensor data of the waist, a body trunk advancing action corresponding to each step action for each of the detected step action groups. Then, the information processing apparatus 100 calculates, for each action group that includes the detected step action group and body trunk advancing action group, an evaluation value that indicates the degree of appearance of each relationship of the plurality of relationships between actions and, based on the calculated evaluation value, determines whether or not the target period is a walking period. In this case, the plurality of relationships includes the first to tenth relationships described later with reference to FIGs. 7 to 16.

Next, an example of the first to tenth relationships between actions used by the information processing apparatus 100 will be described with reference to FIGs. 7 to 16. First, an example of the first relationship between actions will be described with reference to FIG. 7.

FIG. 7 is an explanatory diagram illustrating an example of the first relationship between actions. Here, when the patient walks, there is a property that the step action of the left leg and the step action of the right leg alternately occur one by one. In the example in FIG. 7, step actions 711 to 714 of the left leg and step actions 721 to 724 of the right leg occur alternately one by one.

Thus, the information processing apparatus 100 uses, as the first relationship, "left-right alternation property" in which step actions of the respective legs of the patient occur alternately. The information processing apparatus 100 uses following formula (1) as a calculation formula for calculating the evaluation value of the first relationship.
[Mathematical Formula 1]

$$x_i^{(1)} = I[S_i \neq S_{i+1}] \quad \cdots (1)$$

Here, $x_i^{(1)}$ denotes the evaluation value of the first relationship. A reference i denotes a number assigned to each step action of the patient's two legs in the step action group in the order of appearance, and is a natural number. A reference $S_i$ denotes a variable representing whether an i-th step action is by the left leg or by the right leg. A term I[...] denotes an indicator function, and returns 1 if the condition inside [...] is true, while returning 0 if the condition is false. The condition inside [...] in above formula (1) indicates that the leg of the i-th step action differs from the leg of an (i+1)-th step action immediately after the i-th step action.

Therefore, if the leg of the i-th step action differs from the leg of the (i+1)-th step action immediately after the i-th step action, $x_i^{(1)} = 1$ is established in above formula (1). If the leg of the i-th step action is identical to the leg of the (i+1)-th step action immediately after the i-th step action, $x_i^{(1)} = 0$ is established in above formula (1). In addition, the information processing apparatus 100 may use following formula (2) instead of above formula (1).
[Mathematical Formula 2]

$$x_i^{(1)} = \begin{cases} 1 \ (S_i = right) \\ 0 \ (S_i = left) \end{cases} \quad \cdots (2)$$

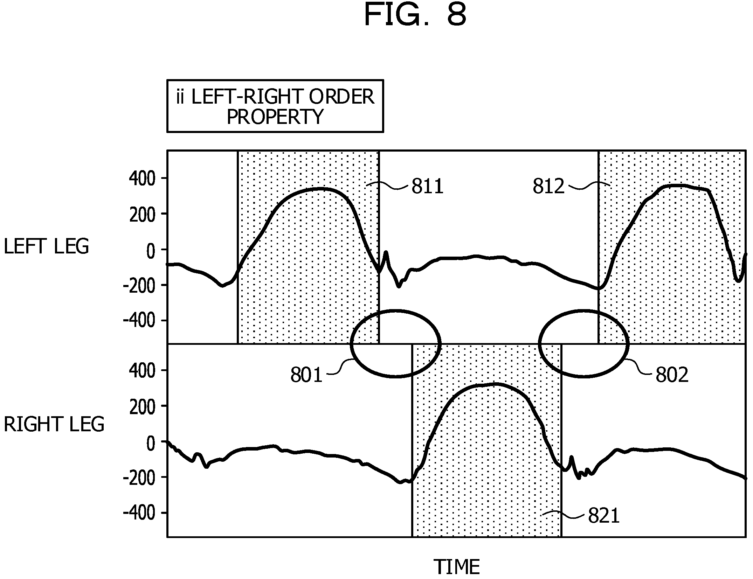

Here, $x_i^{(1)}$ denotes the evaluation value of the first relationship. A reference $S_i$ denotes a variable representing whether the i-th step action is by the left leg or by the right leg. Next, description of FIG. 8 will be made, and an example of the second relationship between actions will be described.

FIG. 8 is an explanatory diagram illustrating an example of the second relationship between actions. Here, when the patient walks, there is a property that one leg of the patient leaves the ground within a predetermined time after the other leg of the patient lands. In the example in FIG. 8, after a step action 811 of the left leg ends and the left leg lands, a step action 821 of the right leg starts and the right leg leaves the ground within a predetermined time 801. Similarly, after the step action 821 of the right leg ends and the right leg lands, a step action 812 of the left leg starts and the left leg leaves the ground within a predetermined time 802.

Thus, the information processing apparatus 100 uses, as the second relationship, "left-right order property" in which the step action of one leg of the patient starts within a predetermined time after the step action of the other leg of the patient ends. The information processing apparatus 100 uses following formula (3) as a calculation formula for calculating the evaluation value of the second relationship.
[Mathematical Formula 3]

$$x_i^{(2)} = I\left[T_i^{(IC)} < T_{i+1}^{(TC)}\right] \quad \cdots (3)$$

Here, $x_i^{(2)}$ denotes the evaluation value of the second relationship. A reference i denotes a number assigned to each step action of the patient's two legs in the step action group in the order of appearance, and is a natural number. References $T^{(TC)}$ and $T^{(IC)}$ represent the start time and end time of the step action, respectively. A term I[...] denotes an indicator function, and returns 1 if the condition inside [...] is true, while returning 0 if the condition is false. The condition inside [...] in above formula (3) indicates that the start time of the (i+1)-th step action immediately after the i-th step action comes after the end time of the i-th step action.

Therefore, if the start time of the (i+1)-th step action immediately after the i-th step action comes after the end time of the i-th step action, $x_i^{(2)} = 1$ is established in above formula (3). If the start time of the (i+1)-th step action immediately after the i-th step action does not come after the end time of the i-th step action, $x_i^{(2)} = 0$ is established in above formula (3).

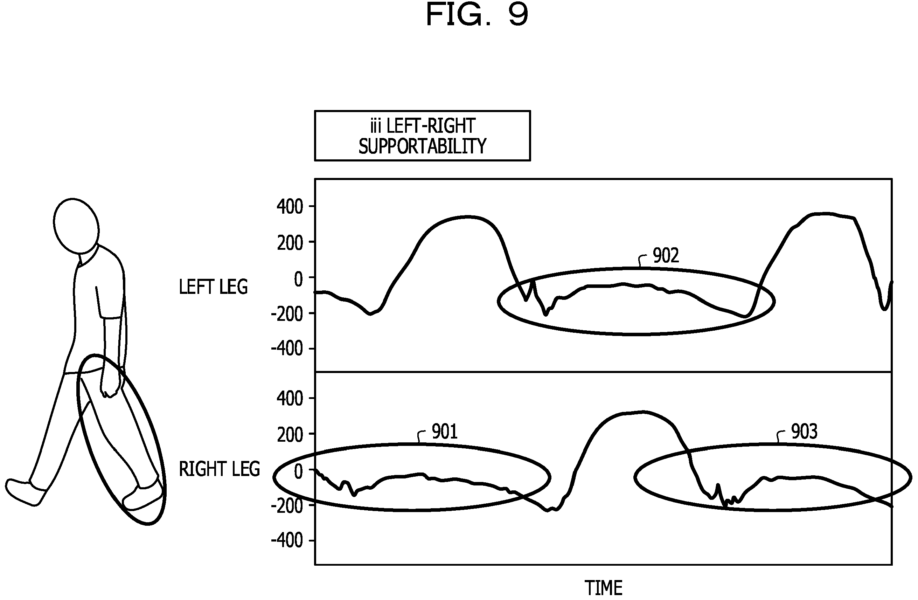

FIG. 9 is an explanatory diagram illustrating an example of the third relationship between actions. Here, when the patient walks, there is a property that, while one leg of the patient is swinging, the other leg of the patient stays on the ground to support the patient's weight in the resting state, and accordingly the supporting leg has a smaller angular velocity. In the example in FIG. 9, while the left leg is being the step action and the left leg is swinging, an angular velocity 901 of the right leg is equal to or less than a threshold. The same applies to an angular velocity 902 of the left leg and an angular velocity 903 of the right leg.

Thus, the information processing apparatus 100 uses, as the third relationship, "left-right supportability" in which, during the step action of one leg of the patient, the angular velocity of the step action of the other leg of the patient falls within a predetermined range. The information processing apparatus 100 uses following formula (4) as a calculation formula for calculating the evaluation value of the third relationship.

[Mathematical Formula 4]

$$x_i^{(3)} = I\left[\alpha < \max_{t \subset \left[T_i^{(TC)}, T_i^{(IC)}\right]} \omega_{inv}(t) < \beta\right] \quad \ldots (4)$$

Here, $x_i^{(3)}$ denotes the evaluation value of the third relationship. A reference i denotes a number assigned to each step action of the patient's two legs in the step action group in the order of appearance, and is a natural number. A reference $\omega_{inv}$ denotes sensor data of not the leg of the i-th step action but the leg not performing the step action, and represents gyro data. A reference t denotes a variable representing time. References $\alpha$ and $\beta$ represent the range of angular velocity that represents the resting state of the leg. References $T^{(TC)}$ and $T^{(IC)}$ represent the start time and end time of the step action, respectively. A term I[...] denotes an indicator function, and returns 1 if the condition inside [...] is true, while returning 0 if the condition is false. The condition inside [...] in above formula (4) indicates that the maximum value of the angular velocity of the leg other than the leg of the i-th step action falls within a predetermined range and this leg is in the resting state.

Therefore, if the maximum value of the angular velocity of the leg other than the leg of the i-th step action from $T^{(TC)}$ to $T^{(IC)}$ falls within a predetermined range from $\alpha$ to $\beta$, and this leg is in the resting state, $x_i^{(3)} = 1$ is established in above formula (4). If the maximum value of the angular velocity of the leg other than the leg of the i-th step action from $T^{(TC)}$ to $T^{(IC)}$ does not fall within the predetermined range from $\alpha$ to $\beta$, and this leg is not in the resting state, $x_i^{(3)} = 0$ is established in above formula (4) .

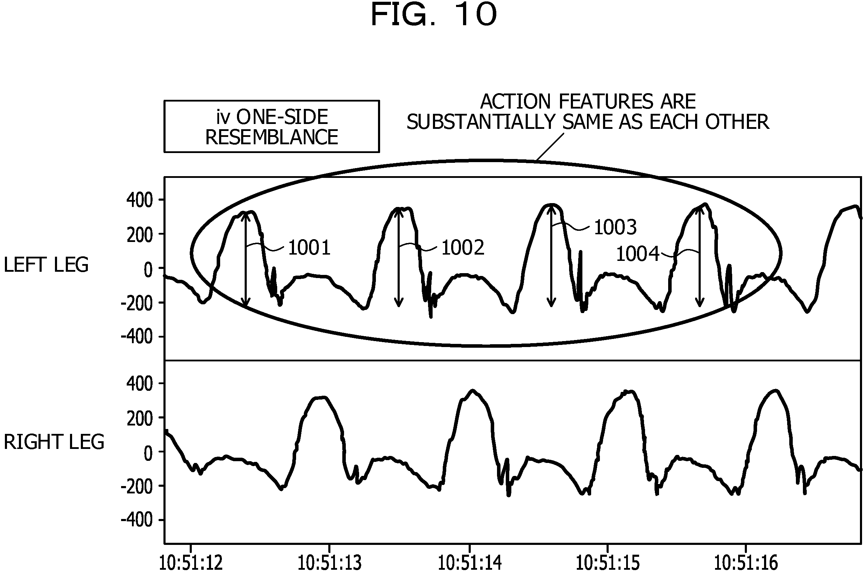

FIG. 10 is an explanatory diagram illustrating an example of the fourth relationship between actions. Here, when the patient walks, there is a property that the angular velocity of the step action of one leg of the patient tends to be constant. In the example in FIG. 10, peak amplitudes 1001 to 1004 of angular velocities of step actions of the left leg are constant. Furthermore, in addition to the peak amplitude, there are cases where peak time is constant. The peak time is a time from a time point when the angular velocity reaches a local minimum value to a time point when the angular velocity reaches a local minimum value following a time point when reaching a local maximum value, before and after the step action.

Thus, the information processing apparatus 100 uses, as the fourth relationship, "one-side resemblance" in which feature quantities of a plurality of step actions of one leg of the patient resemble each other. The information processing apparatus 100 uses following formulas (5) and (6) as calculation formulas for calculating the evaluation value of the fourth relationship.

[Mathematical Formula 5]

$$x_i^{(4A)} = |p_i - \overline{p}|/\overline{p} \quad where \quad p_i = \max_{t \subset \left[T_i^{(TC)}, T_i^{(IC)}\right]} \omega(t) \quad \ldots (5)$$

Here, $x_i^{(4A)}$ denotes the evaluation value of the fourth relationship. A reference i denotes a number assigned to each step action of the patient's two legs in the step action group in the order of appearance, and is a natural number. A reference p represents the peak amplitude of the step action. A symbol with a bar at the top of the reference p represents the average of the peak amplitudes of the step actions in the step action group. A reference $\omega$ denotes sensor data of the leg of the i-th step action and represents gyro data. References $T^{(TC)}$ and $T^{(IC)}$ represent the start time and end time of the step action, respectively.

Therefore, if the peak amplitude of the i-th step action of one leg of the patient resembles the peak amplitude of another step action of the same leg, $x_i^{(4A)}$ takes a smaller value in above formula (5). If the peak amplitude of the i-th step action of one leg of the patient does not resemble the peak amplitude of another step action of the same leg, $x_i^{(4A)}$ takes a larger value in above formula (5).

[Mathematical Formula 6]

$$x_i^{(4B)} = |q_i - \overline{q}| / \overline{q} \ \ where \ \ q_i = T_i^{(IC)} - T_i^{(TC)} \quad ... (6)$$

Here, $x_i^{(4B)}$ denotes the evaluation value of the fourth relationship. A reference i denotes a number assigned to each step action of the patient's two legs in the step action group in the order of appearance, and is a natural number. A reference q represents the peak time of the step action. A symbol with a bar at the top of the reference q represents the average value of peak times of the step actions in the step action group. References $T^{(TC)}$ and $T^{(IC)}$ represent the start time and end time of the step action, respectively.

Therefore, if the peak time of the i-th step action of one leg of the patient resembles the peak time of another step action of the same leg, $x_i^{(4B)}$ takes a smaller value in above formula (6). If the peak time of the i-th step action of one leg of the patient does not resemble the peak time of another step action of the same leg, $x_i^{(4B)}$ takes a larger value in above formula (6).

In addition, the information processing apparatus 100 may use, as the peak amplitude p, an angular velocity at the start time of the step action or an angular velocity at the end time of the step action. The information processing apparatus 100 may use, as the peak time q, a time from the start time of the step action to a time point when the angular velocity of the step action reaches a local maximum, or a time from a time point when the angular velocity of the step action reaches a local maximum to the end time of the step action. In addition, the information processing apparatus 100 may use, as the peak time q, a time from the end time of the step action to the start time of the next step action.

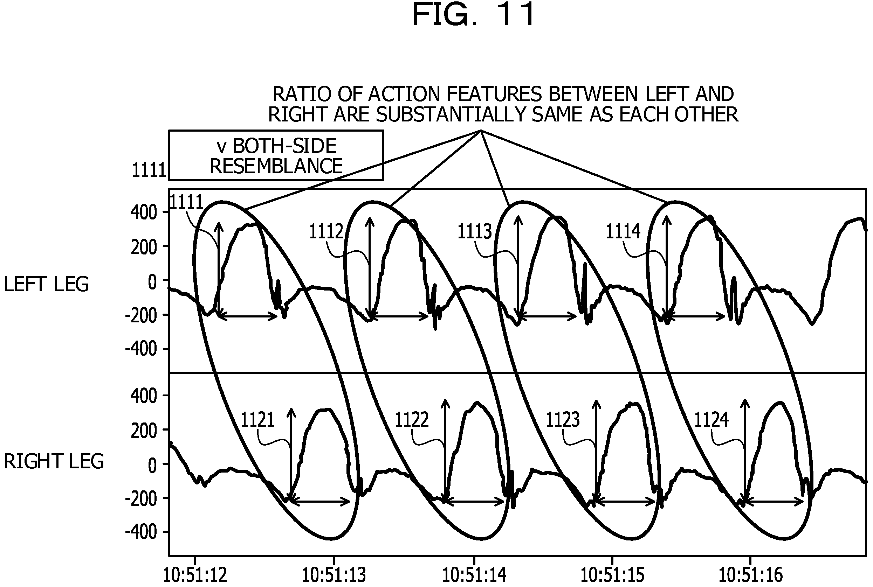

FIG. 11 is an explanatory diagram illustrating an example of the fifth relationship between actions. Here, when the patient walks, there is a property that the ratio of the feature quantities of the step actions between the left and right leg of the patient tends to be constant. In the example in FIG. 11, the ratios between peak amplitudes 1111 to 1114 of angular velocities of step actions of the left leg and peak amplitudes 1121 to 1124 of angular velocities of step actions of the right leg are constant. Furthermore, in addition to the peak amplitude, there are cases where the ratio of peak times is constant.

Thus, the information processing apparatus 100 uses, as the fifth relationship, "both-side resemblance" in which feature quantities of step actions of both the legs of the patient resemble each other. The information processing apparatus 100 uses following formulas (7) and (8) as calculation formulas for calculating the evaluation value of the fifth relationship.

[Mathematical Formula 7]

$$x_i^{(5A)} = |r_i - \overline{r}| / \overline{r} \ \ where \ \ r_i = f_L(p_i, p_{i+1}) / f_R(p_i, p_{i+1}) \quad ... (7)$$

Here, $x_i^{(5A)}$ denotes the evaluation value of the fifth relationship. A reference i denotes a number assigned to each step action of the patient's two legs in the step action group in the order of appearance, and is a natural number. A reference p represents the peak amplitude of the step action. A reference $r_i$ represents the ratio of peak amplitudes of consecutive step actions of the left and right legs. A symbol with a bar at the top of the reference $r_i$ represents the average value of the ratios of peak amplitudes of step actions of the left and right legs in the step action group. A term $f_L(a,b)$ is a function that selects, from a and b, a feature quantity related to the left leg, for example, the peak amplitude of the left leg. A term $f_R(a,b)$ is a function that selects, from a and b, a feature quantity related to the right leg, for example, the peak amplitude of the right leg.

Therefore, if the ratio of the peak amplitudes between the i-th step action and the (i+1)-th step action, which are the step actions of the left and right legs, resembles the average value of the ratios of peak amplitudes of step actions of the left and right legs, $x_i^{(5A)}$ takes a smaller value in above formula (7). If the ratio of the peak amplitudes between the i-th step action and the (i+1)-th step action, which are the step actions of the left and right legs, does not resemble the average value of the ratios of peak amplitudes of step actions of the left and right legs, $x_i^{(5A)}$ takes a larger value in above formula (7).
[Mathematical Formula 8]

$$x_i^{(5B)} = |s_i - \bar{s}|/\bar{s} \ where \ s_i = f_L(q_i, q_{i+1})/f_R(q_i, q_{i+1}) \quad \dots (8)$$

Here, $x_i^{(5B)}$ denotes the evaluation value of the fifth relationship. A reference i denotes a number assigned to each step action of the patient's two legs in the step action group in the order of appearance, and is a natural number. A reference q represents the peak time of the step action. A reference si represents the ratio of peak times of step actions of the left and right legs. A symbol with a bar at the top of the reference $r_i$ represents the average value of the ratios of peak times of step actions of the left and right legs in the step action group. A term $f_L(a,b)$ is a function that selects, from a and b, a feature quantity related to the left leg, for example, the peak time of the left leg. A term $f_R(a,b)$ is a function that selects, from a and b, a feature quantity related to the right leg, for example, the peak time of the right leg.

Therefore, if the ratio of the lengths between the i-th step action and the (i+1)-th step action, which are the step actions of the left and right legs, resembles the average value of the ratios of peak times of step actions of the left and right legs, $x_i^{(5B)}$ takes a smaller value in above formula (8). If the ratio of the lengths between the i-th step action and the (i+1)-th step action, which are the step actions of the left and right legs, does not resemble the average value of the ratios of peak times of step actions of the left and right legs, $x_i^{(5B)}$ takes a larger value in above formula (8).

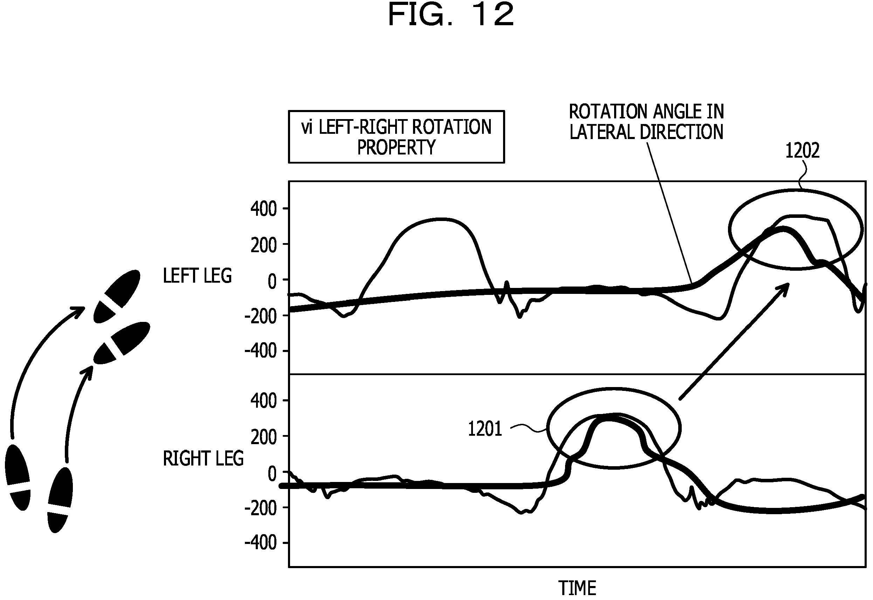

FIG. 12 is an explanatory diagram illustrating an example of the sixth relationship between actions. Here, when the patient walks, there is a property that the rotation direction of the step action of the left leg of the patient coincides with the rotation direction of the step action of the right leg of the patient. The example in FIG. 12 illustrates gyro data on the traverse plane of each leg. In addition, the rotation angle in the lateral direction in FIG. 12 is a value calculated by integrating the angular velocity on the traverse plane in the time direction. In the example in FIG. 12, a rotation angle 1201 of the right leg in the lateral direction indicates right rotation and, similarly, a rotation angle 1202 of the left leg in the lateral direction indicates right rotation; accordingly, the rotation directions coincide with each other.

Thus, the information processing apparatus 100 uses, as the sixth relationship, "left-right rotation property" in which the rotation directions of step actions of the respective legs of the patient are identical to each other. The information processing apparatus 100 uses following formula (9) as a calculation formula for calculating the evaluation value of the sixth relationship.

[Mathematical Formula 9]

$$x_i^{(6)} = I\left[\left(sgn(R_{i-1}) = sgn(R_i)\right) \cup \left(sgn(R_{i+1}) = sgn(R_i)\right)\right] \quad \dots (9)$$

Here, $x_i^{(6)}$ denotes the evaluation value of the sixth relationship. A reference i denotes a number assigned to each step action of the patient's two legs in the step action group in the order of appearance, and is a natural number. A reference Ri represents the rotation amount of the i-th step action. The rotation amount is, for example, an integral value of the angular velocity. A term sgn(...) denotes a sign function, and returns "1" if the contents of (...) indicate 0 or more, while returning "-1" if the contents of (...) indicate 0 or less. A term I[...] denotes an indicator function, and returns 1 if the condition inside [...] is true, while returning 0 if the condition is false. The condition inside [...] in above formula (9) indicates that the rotation direction of the i-th step action is identical to the rotation direction of the immediately following (i+1)-th step action or the immediately preceding (i-1)-th step action.

Therefore, if the rotation amount of the i-th step action exceeds a threshold, and the rotation direction of the i-th step action is identical to the rotation direction of the immediately following (i+1)-th step action or the immediately preceding (i-1)-th step action, $x_i^{(6)} = 1$ is established in above formula (9). If the rotation direction of the i-th step action is not identical to the rotation direction of the immediately following (i+1)-th step action or the immediately preceding (i-1)-th step action, $x_i^{(6)} = 0$ is established in above formula (9).

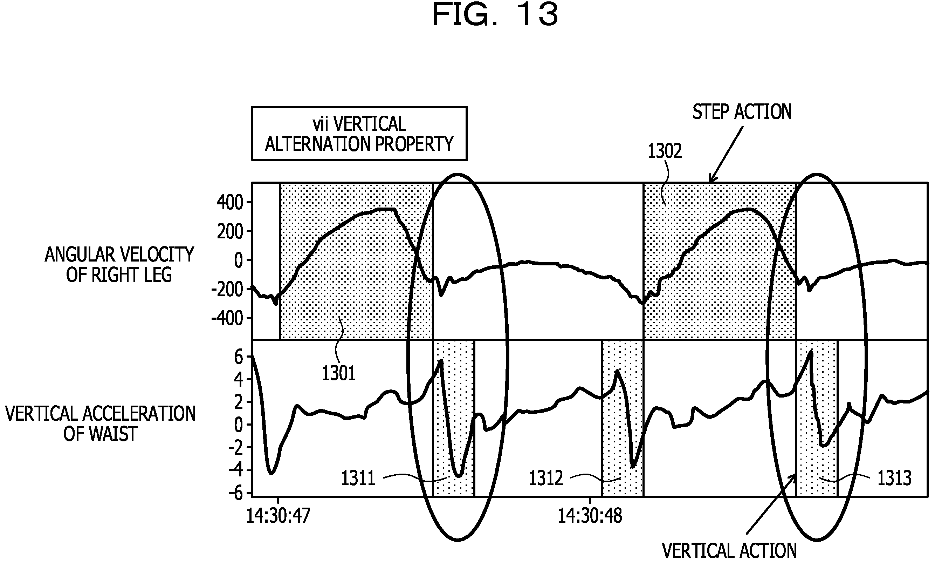

FIG. 13 is an explanatory diagram illustrating an example of the seventh relationship between actions. Here, when the patient walks, there is a property that a large vertical acceleration is produced also in the patient's waist before and after each leg of the patient lands. In the example in FIG. 13, vertical actions 1311 and 1313 of the waist are produced immediately after step actions 1301 and 1302 of the right leg, as the body trunk advancing actions.

Besides, there is a property that a large vertical acceleration is produced also in the patient's waist before and after each leg of the patient leaves the ground. In the example in FIG. 13, a vertical action 1312 of the waist is produced immediately before the step action 1302 of the right leg, as the body trunk advancing action.

Thus, the information processing apparatus 100 uses, as the seventh relationship, "vertical alternation property" in which the step action of the body trunk of the patient in the vertical direction occurs in a range of predetermined time before and after the step action of each leg of the patient ends. The information processing apparatus 100 uses following formula (10) as a calculation formula for calculating the evaluation value of the seventh relationship.
[Mathematical Formula 10]

$$x_i^{(7)} = I\left[\left(\max_{t\subset\left[T_i^{(IC)}-\tau,\,T_i^{(IC)}+\tau\right]} acc_z(t) - \min_{t\subset\left[T_i^{(IC)}-\tau,\,T_i^{(IC)}+\tau\right]} acc_z(t)\right) > \gamma\right] \quad \ldots (10)$$

Here, $x_i^{(7)}$ denotes the evaluation value of the seventh relationship. A reference i denotes a number assigned to each step action of the patient's two legs in the step action group in the order of appearance, and is a natural number. A reference $acc_z$ represents vertical acceleration data of the patient's waist. A reference $\gamma$ denotes a threshold. A reference $T^{(IC)}$ represents the end time of the step action. A reference $\tau$ represents a predetermined range before and after the end time. A term I[...] denotes an indicator function, and returns 1 if the condition inside [...] is true, while returning 0 if the condition is false. The condition inside [...] in above formula (10) indicates that a change in vertical acceleration of the patient's waist is equal to or more than the threshold, near the end time of the i-th step action.

Therefore, if a change in vertical acceleration of the waist of the patient is equal to or more than the threshold near the end time of the i-th step action of the leg of the same patient, $x_i^{(7)} = 1$ is established in above formula (10). If a change in vertical acceleration of the waist of the patient is not equal to or more than the threshold near the end time of the i-th step action of the leg of the same patient, $x_i^{(7)} = 0$ is established in above formula (10).

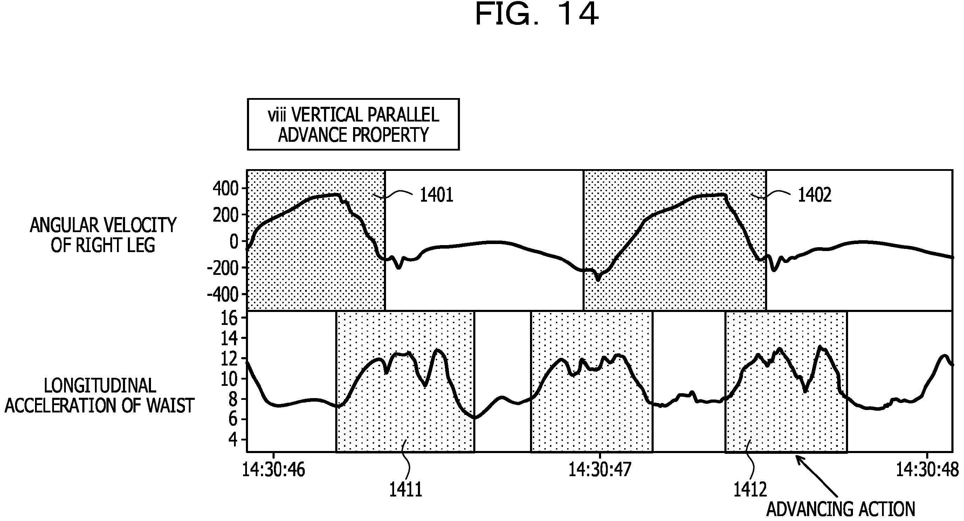

FIG. 14 is an explanatory diagram illustrating an example of the eighth relationship between actions. Here, when the patient walks, there is a property that, when each leg of the patient moves forward, the waist of the patient also moves forward similarly. In the example in FIG. 14, longitudinal actions 1411 and 1412 of the waist occur immediately after step actions 1401 and 1402 of the right leg, as body trunk advancing actions, and the integral value of the longitudinal acceleration of the waist is equal to or more than a threshold.

Thus, the information processing apparatus 100 uses, as the eighth relationship, "vertical parallel advance property" in which the step action of the body trunk of the patient in the advance direction occurs during the step action of each leg of the patient. The information processing apparatus 100 uses following formula (11) as a calculation formula for calculating the evaluation value of the eighth relationship.
[Mathematical Formula 11]

$$x_i^{(8)} = I\left[\sum_{t\subset\left[T_i^{(IC)}-Ts,\,T_i^{(IC)}+Te\right]} acc_x(t) > \gamma\right] \quad \ldots (11)$$

Here, $x_i^{(8)}$ denotes the evaluation value of the eighth relationship. A reference i denotes a number assigned to each step action of the patient's two legs in the step action group in the order of appearance, and is a natural number. A reference accx represents longitudinal acceleration data of the patient's waist. References Ts and Te denote time parameters that designate a period during which the waist is moving forward. References $T^{(TC)}$ and $T^{(IC)}$ represent the start time and end time of the step action, respectively. A term I[...] denotes an indicator function, and returns 1 if the condition inside [...] is true, while returning 0 if the condition is false. The condition inside [...] in above formula (11) indicates that the integral value of the longitudinal acceleration of the patient's waist in a range from a point before Ts to a point after Te of the end time of the i-th step action among the step actions of the two legs of the patient is larger than the threshold.

Therefore, if the integral value of the longitudinal acceleration of the patient's waist is larger than the threshold in a range from a point before Ts to a point after Te of the end time of the i-th step action, $x_i^{(8)} = 1$ is established in above formula (11). If the integral value of the longitudinal acceleration of the patient's waist is not larger than the threshold in a range from a point before Ts to a point after Te of the end time of the i-th step action, $x_i^{(8)} = 0$ is established in above formula (11).

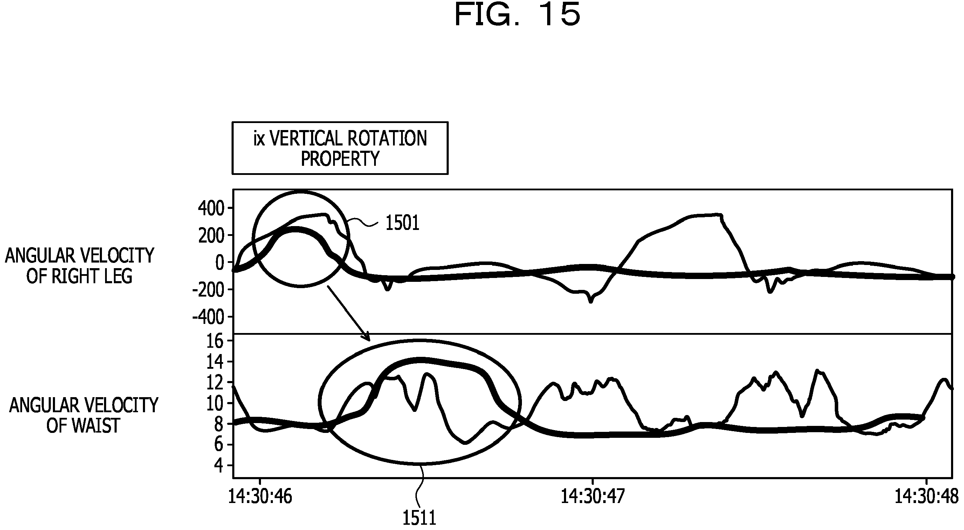

FIG. 15 is an explanatory diagram illustrating an example of the ninth relationship between actions. Here, when the patient walks, there is a property that the rotation direction of each leg of the patient coincides with the rotation direction of the waist of the patient. The example in FIG. 15 illustrates gyro data of the right leg and the waist on the traverse plane. In the example in FIG. 15, the rotation direction in a step action 1501 of the right leg coincides with the rotation direction in a body trunk advancing action 1511 of the waist.

Thus, the information processing apparatus 100 uses, as the ninth relationship, "vertical rotation property" in which the rotation direction of the step action of each leg of the patient is identical to the rotation direction of the step action of the body trunk of the patient after the step action of each leg of the patient ends. The information processing apparatus 100 uses following formula (12) as a calculation formula for calculating the evaluation value of the ninth relationship.

[Mathematical Formula 12]

$$x_i^{(9)} = I\big[sig\big(R_i^{leg}\big) = sig\big(R_i^{waist}\big)\big] \quad \dots (12)$$

$$where \; R_i^{leg} = \sum_{1 \subset \left[T_i^{(IC)}-Ts, T_i^{(IC)}+Te\right]} \omega_Y^{leg}(t), \; R_i^{waist} = \sum_{1 \subset \left[T_i^{(IC)}-Ts, T_i^{(IC)}+Te\right]} \omega_Y^{waist}(t)$$

Here, $x_i^{(9)}$ denotes the evaluation value of the ninth relationship. A reference $R_i^{leg}$ represents the rotation amount of the right or left leg. A reference $R_i^{waist}$ represents the rotation amount of the waist. A reference i denotes a number assigned to each step action of the patient's two legs in the step action group in the order of appearance, and is a natural number. A reference $\omega_Y^{waist}$ represents gyro data of the waist on the traverse plane. A reference $\omega_Y^{leg}$ represents gyro data of the right or the left leg on the traverse plane. References $T^{(TC)}$ and $T^{(IC)}$ represent the start time and end time of the step action, respectively. References Ts and Te denote time parameters that designate a timing when the upper body advances. A term I[...] denotes an indicator function, and returns 1 if the condition inside [...] is true, while returning 0 if the condition is false. The condition inside [...] in above formula (12) indicates that the rotation direction of the waist coincides with the rotation direction of the leg in the i-th step action.

Therefore, if the rotation direction of the waist coincides with the rotation direction of the leg in the i-th step action, $x_i^{(9)} = 1$ is established in above formula (12). If the rotation direction of the waist does not coincide with the rotation direction of the leg in the i-th step action, $x_i^{(9)} = 0$ is established in above formula (12).

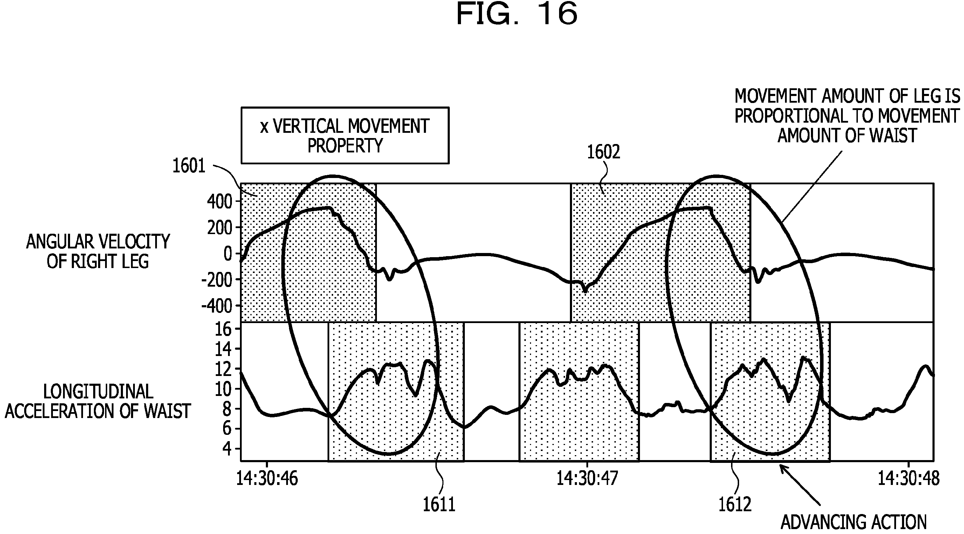

FIG. 16 is an explanatory diagram illustrating an example of the tenth relationship between actions. Here, when the patient walks, there is a property that the movement amount of the leg of the patient resembles the movement amount of the waist of the patient. In the example in FIG. 16, the movement amounts of step actions 1601 and 1602 of the right leg resemble the movement amounts of body trunk advancing actions 1611 and 1612 of the waist.

Thus, the information processing apparatus 100 uses, as the tenth relationship, "vertical movement property" in which the movement amount indicated by the step action of each leg of the patient resembles the movement amount indicated by the step action of the body trunk of the patient. The information processing apparatus 100 uses following formula (13) as a calculation formula for calculating the evaluation value of the tenth relationship.

[Mathematical Formula 13]

$$x_i^{(10)} = I[\eta_{min} < (v_i \,/\, u_i) < \eta_{max}] \quad \dots (13)$$

Here, $x_i^{(10)}$ denotes the evaluation value of the tenth relationship. A reference i denotes a number assigned to each step action of the patient's two legs in the step action group in the order of appearance, and is a natural number. A reference v represents the movement amount of the leg in the step action. A reference u represents the movement amount of the waist in the body trunk advancing action. References $\eta_{min}$ and $\eta_{max}$ denote constants representing the range of the ratio between the movement amount of the patient's leg and the movement amount of the patient's waist. A term I[...] denotes an indicator function, and returns 1 if the condition inside [...] is true, while returning 0 if the condition is false. The condition inside [...] in above formula (13) indicates that the ratio between the movement amount of the patient's leg and the movement amount of the patient's waist in the i-th step action falls within the range of a threshold.

Therefore, if the ratio between the movement amount of the patient's leg and the movement amount of the patient's waist in the i-th step action falls within the range of the threshold, $x_i^{(10)} = 1$ is established in above formula (13). If the ratio between the movement amount of the patient's leg and the movement amount of the patient's waist in the i-th step action falls outside the range of the threshold, $x_i^{(10)} = 0$ is established in above formula (13).

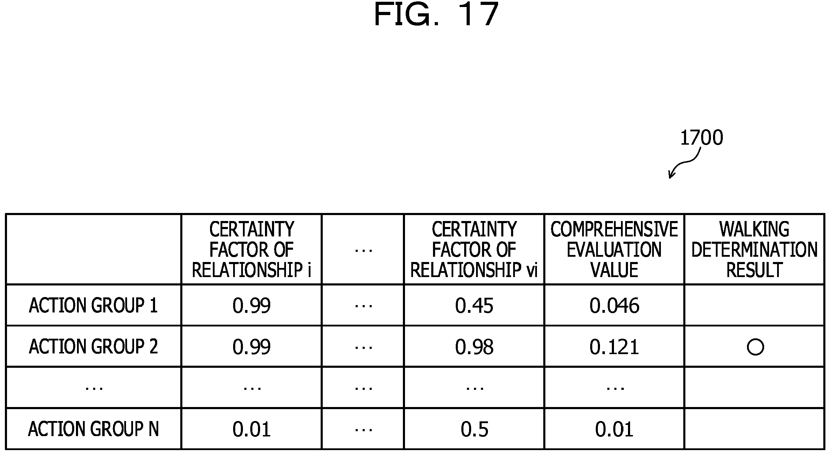

FIG. 17 is an explanatory diagram illustrating an example in which the information processing apparatus 100 determines whether or not the target period is a walking period. In FIG. 17, the information processing apparatus 100 calculates, for each step action group, the average values and the like of the first to tenth relationships calculated using above formulas (1) to (13) and the like, as certainty factors of the first to tenth relationships. The information processing apparatus 100 performs multiplication by the calculated certainty factor to calculate a comprehensive evaluation value that indicates the likeliness to the walking period of the target period. The information processing apparatus 100 stores the certainty factor and the comprehensive evaluation value using a result management table 1700.

Next, the information processing apparatus 100 specifies the maximum value from among the comprehensive evaluation values calculated for the respective step action groups. Then, the information processing apparatus 100 determines whether or not the specified maximum value is equal to or more than a threshold. Here, if the maximum value is equal to or more than the threshold, the information processing apparatus 100 determines that the target period is a walking period. On the other hand, if the maximum value is less than the threshold, the information processing apparatus 100 determines that the target period is not a walking period. The information processing apparatus 100 stores the result of determination using the result management table 1700.

When the information processing apparatus 100 determines that the target period is a walking period, the information processing apparatus 100 may use a step action group from which the maximum value has been calculated, as a step action group that most reflects the way of walking of the patient. Then, the information processing apparatus 100 calculates and outputs a predetermined feature quantity in the target period, using the step action group that most reflects the way of walking of the patient.

(First Specific Example in which Information Processing Apparatus 100 Specifies Walking Period)

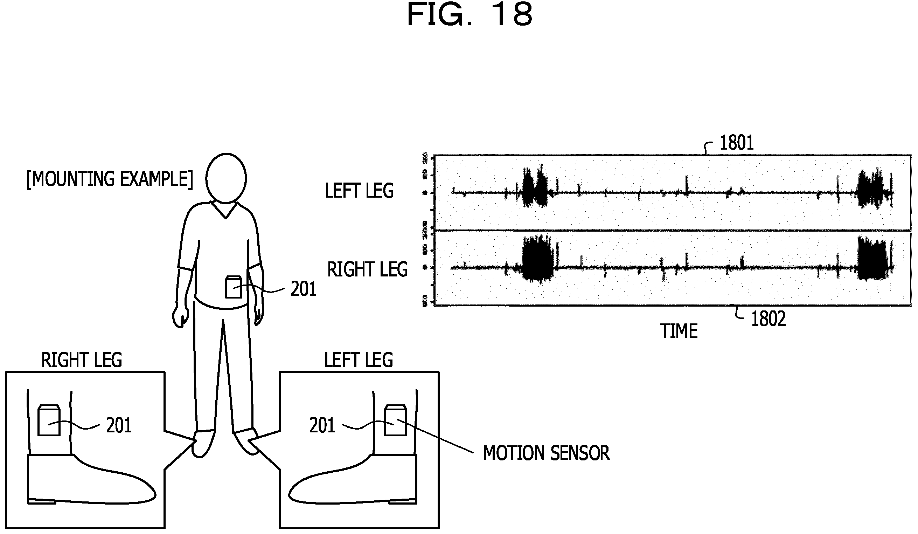

Next, a first specific example in which the information processing apparatus 100 specifies a walking period will be described with reference to FIGs. 18 to 26.

FIGs. 18 to 26 are explanatory diagrams illustrating the first specific example in which the information processing apparatus 100 specifies a walking period. In FIG. 18, a patient mounts the measuring instruments 201 on his/her right leg, left leg, and waist. The measuring instruments 201 measure the angular velocity and acceleration at regions of the patient where the measuring instruments 201 are mounted, and generate sensor data 1801 and sensor data 1802 and the like in association with measurement values and measurement times. The information processing apparatus 100 receives the sensor data 1801, the sensor data 1802, and the like generated by the measuring instruments 201. Next, description of FIGs. 19 and 20 will be made, and the contents of measurement by the measuring instruments 201 will be described.

As illustrated in FIG. 19, when measuring the angular velocity, the measuring instruments 201 measure, for example, the angular velocity on the sagittal plane, the angular velocity on the traverse plane, and the angular velocity on the coronal plane. With this configuration, for example, the measuring instruments 201 generate, as sensor data, gyro data in which angular velocities on the sagittal plane are arranged in time series, gyro data in which angular velocities on the traverse plane are arranged in time series, and gyro data in which angular velocities on the coronal plane are arranged in time series.

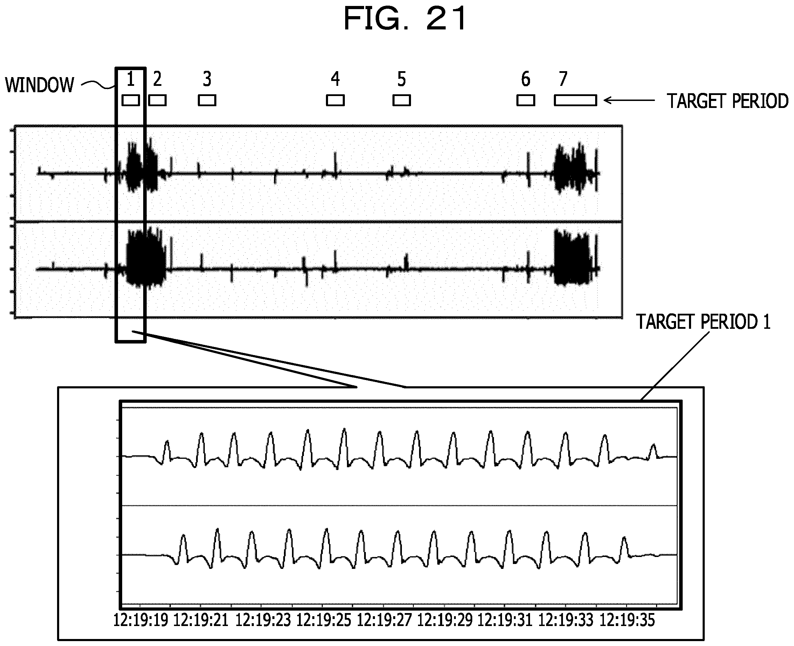

As illustrated in FIG. 20, when measuring the acceleration, the measuring instruments 201 measure, for example, the vertical acceleration, the lateral acceleration, and the longitudinal acceleration. With this configuration, for example, the measuring instruments 201 generate, as sensor data, acceleration data in which vertical accelerations are arranged in time series, acceleration data in which lateral accelerations are arranged in time series, and acceleration data in which longitudinal accelerations are arranged in time series. Next, description of FIG. 21 will be made.

In FIG. 21, the information processing apparatus 100 specifies a non-stationary period in which the patient is not in a stationary state, as a target period to be determined as to whether or not the specified non-stationary period is a walking period, using the received gyro data and acceleration data.

In specifying the target period, for example, the information processing apparatus 100 shifts a window having a window width W by a shift width (W/2) from the same time point in both of the gyro data of the left leg and the gyro data of the right leg. The window indicates a period of predetermined length. Every time the window is shifted, the information processing apparatus 100 specifies segment data corresponding to the shifted window from the gyro data of the left leg and the gyro data of the right leg.

In specifying the target period, every time the window is shifted, the information processing apparatus 100 determines whether or not an average value Mg and a variance Vg of the angular velocity exceed predetermined values TH_Mg and TH_Vg, respectively, in the segment data corresponding to the shifted window. When segment data corresponding to a certain window is determined to have the average value Mg and the variance Vg exceeding the predetermined values TH_Mg and TH_Vg, the information processing apparatus 100 specifies a period indicated by the certain window as a non-stationary period in which the patient is not in a stationary state.

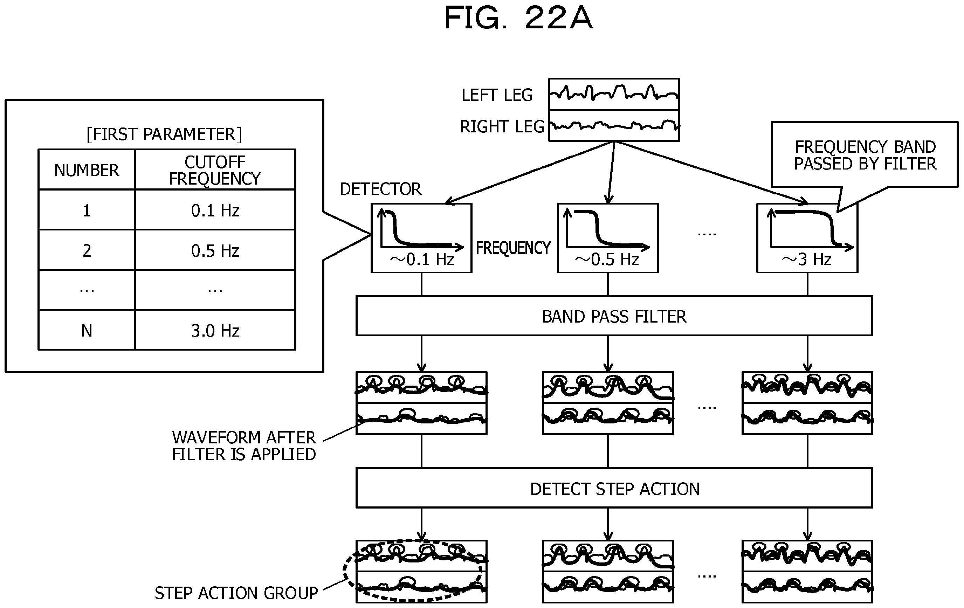

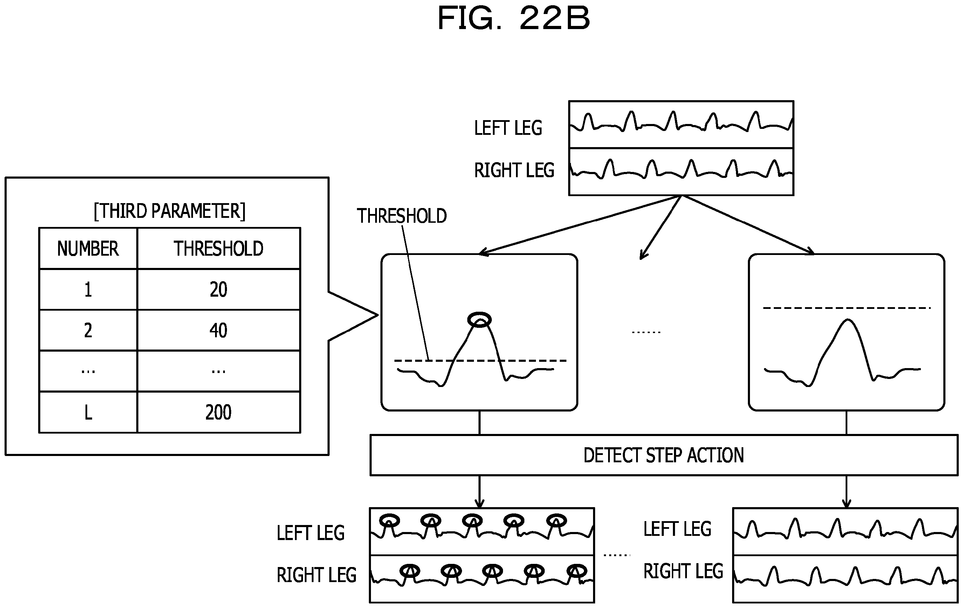

In specifying the target period, if the specified non-stationary periods include two or more consecutive non-stationary periods, the information processing apparatus 100 merges the two or more consecutive non-stationary periods, and specifies the merged non-stationary periods as one non-stationary period. The information processing apparatus 100 sets the specified non-stationary period as a target period. Next, description of FIGs. 22A and 22B will be made.

In FIG. 22A, the information processing apparatus 100 applies each band pass filter of a plurality of band pass filters to sensor data for the target period, using N first parameters. The first parameter defines a frequency band for which the band pass filter allows passing, and is expressed by, for example, a cutoff frequency indicating what frequency component is to be attenuated. With this procedure, the information processing apparatus 100 can cope with a case where the way of walking is different for each patient, and the cycle of step action of each leg is different for each patient, and a case where the sensor data contains a noise signal, and can facilitate the detection of the step action of each leg.

Next, the information processing apparatus 100 detects a step action group that includes step actions of the respective legs of the patient, for a case where each band pass filter is applied. The information processing apparatus 100 detects, for example, that the angular velocity of the left leg has exceeded the threshold and reached a local maximum value for a case where each band pass filter is applied, and detects the step action of the left leg. In addition, the information processing apparatus 100 detects, for example, that the angular velocity of the right leg has exceeded the threshold and reached a local maximum value for a case where each band pass filter is applied, and detects the step action of the right leg. Next, description of FIG. 22B will be made, and an example in which the information processing apparatus 100 facilitates the detection of the step action will be described.

In FIG. 22B, the information processing apparatus 100 prepares, as third parameters, a plurality of thresholds that can be set as a threshold used when detecting the local maximum value. In detecting the local maximum value, the information processing apparatus 100 may detect a plurality of step action groups by applying each threshold of the plurality of thresholds to the sensor data or the result of applying the band filter to the sensor data. With this procedure, the information processing apparatus 100 can cope with a case where the way of walking is different for each patient, and the swing speed of the step action is different for each patient, and can facilitate the detection of the step action of each leg.

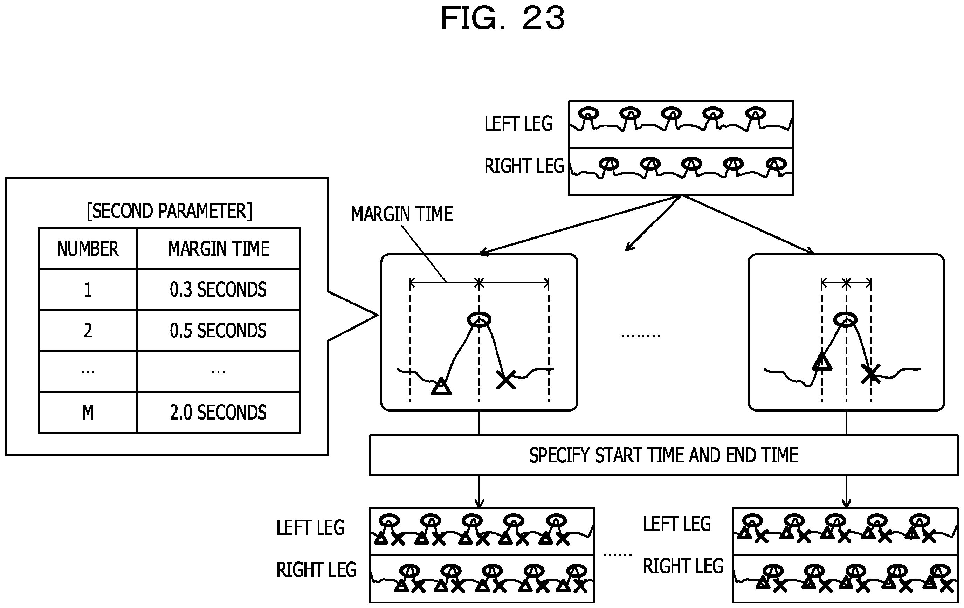

The information processing apparatus 100 stores, as an action point of the step action of each leg, a time point when the angular velocity of each leg reached a local maximum value, and stores the step action of each leg by the action point. The information processing apparatus 100 detects a step action group that includes the detected step actions of the respective legs. In addition, the information processing apparatus 100 may detect a body trunk advancing action of the waist corresponding to the step action of each leg. Next, description of FIG. 23 will be made.

In FIG. 23, the information processing apparatus 100 sets the time length upper limit of the step action using M second parameters for each of the detected step action groups, and specifies the start time and end time of the step action to be used to calculate the evaluation values of the plurality of relationships. The start time to be specified is, for example, a timing when the heel or toe or sole leaves the ground, and the end time to be specified is, for example, a timing when the heel or toe or sole lands on the ground. The second parameter indicates the time length upper limit of the step action, and indicates a length from a time point before the action point by a margin time to a time point after the action point by a margin time.

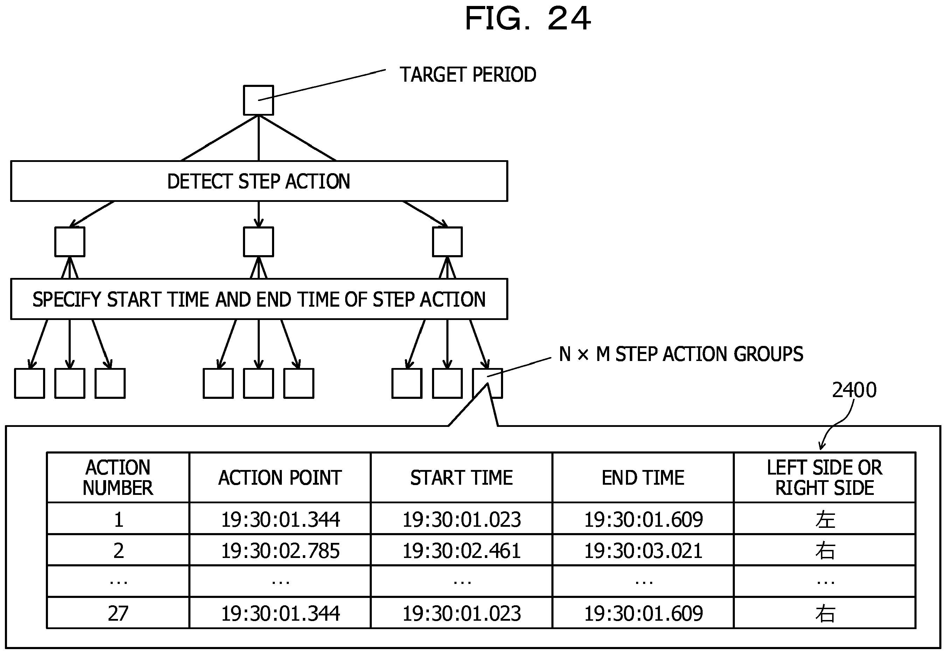

For example, the information processing apparatus 100 specifies, as the start time, a time point when the angular velocity reached the minimum value between the action point of the step action and a point before the action point by the second parameter. For example, the information processing apparatus 100 specifies, as the end time, a time point when the angular velocity reached the minimum value between the action point of the step action and a point after the action point by the second parameter. Next, description of FIG. 24 will be made.

In FIG. 24, the information processing apparatus 100 detects N × M step action groups using N band pass filters in detecting the step action and using M time length upper limits of the step action in specifying the start time and end time of the step action. The information processing apparatus 100 assigns, for each step action group, action numbers to step actions of the respective legs included in each step action group in the order of appearance.

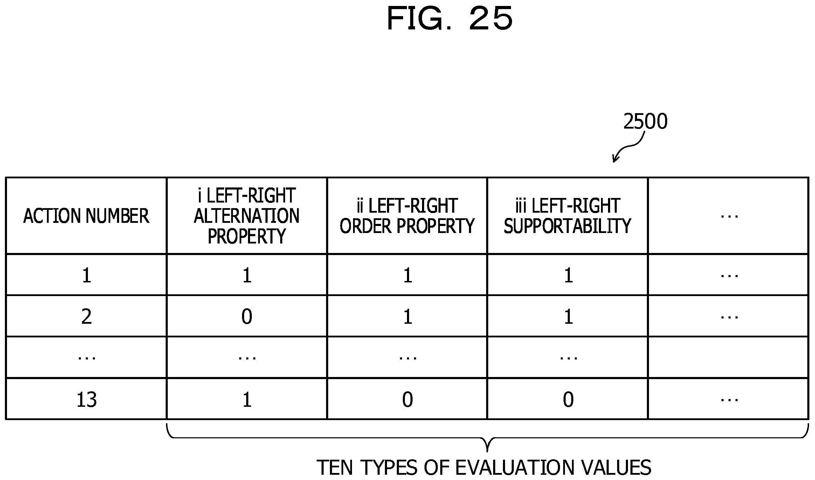

Then, the information processing apparatus 100 stores the step action of each leg using a leg action management table 2400 in association with the action number, the action point, the start time, the end time, and which leg performs the step action. The information processing apparatus 100 may store a body trunk advancing action of the waist corresponding to the step action of each leg using a body trunk advancing action management table. Next, description of FIG. 25 will be made.

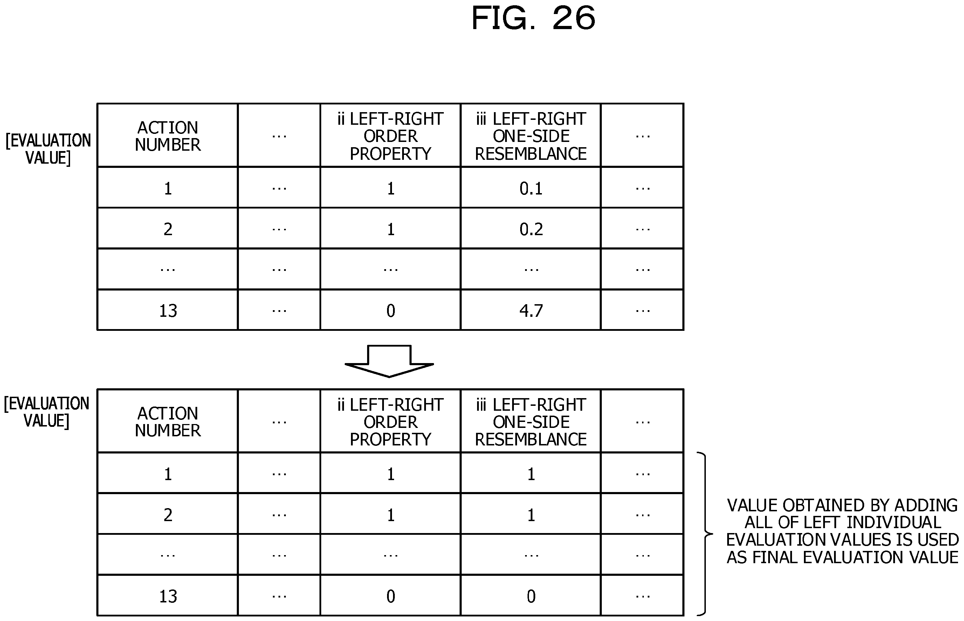

In FIG. 25, the information processing apparatus 100 calculates evaluation values of the first to the tenth relationships for each step action based on the step action group. The information processing apparatus 100 stores the evaluation values of the first to tenth relationships calculated for each step action using an evaluation value management table 2500 in association with the action number of each step action. Next, description of FIG. 26 will be made.

In FIG. 26, the information processing apparatus 100 recalculates the evaluation values of the first to tenth relationships according to a unified criterion. For example, the evaluation value of the second relationship has a binary value, and 1 indicates the likeliness to the walking motion, while 0 indicates the non-likeliness to the walking motion. Meanwhile, the evaluation value of the third relationship has a continuous value, and a value closer to 0 indicates more likeliness to the walking motion, while a larger value indicates less likeliness to the walking motion.

For this reason, the information processing apparatus 100 converts the evaluation value of the third relationship into an evaluation value having a binary value in which 1 indicates the likeliness to the walking motion, and 0 indicates the non-likeliness to the walking motion. For example, the information processing apparatus 100 converts the evaluation value of the third relationship to 1 if the evaluation value is less than a threshold, and converts the evaluation value to 0 if the evaluation value is equal to or more than the threshold.

Then, the information processing apparatus 100 adds up the evaluation values of the first to tenth relationships, and calculates a comprehensive evaluation value. The information processing apparatus 100 determines that the target period is a walking period if the calculated comprehensive evaluation value is equal to or more than a threshold. With this procedure, the information processing apparatus 100 can improve the accuracy of specifying the walking period.

(Second Specific example in which Information Processing Apparatus 100 Specifies Walking Period)

Next, a second specific example in which the information processing apparatus 100 specifies a walking period will be described with reference to FIGs. 27 to 33.

FIGs. 27 to 33 are explanatory diagrams illustrating the second specific example in which the information processing apparatus 100 specifies a walking period. Here, it is conceivable that the target period includes both of a walking period in which a walking motion was performed by the patient and a non-walking period in which no walking motion was performed by the patient. Therefore, the information processing apparatus 100 determines which partial period of the target period is a walking period, based on the evaluation values calculated for the first to the tenth relationships.

As illustrated in FIG. 27, the information processing apparatus 100 applies a hidden Markov model (HMM) that labels the state of each step action as "walking" or "non-walking" in order to distinguish between the walking period and the non-walking period.

The HMM is a state transition model that assumes that the observed feature quantity is generated based on the state-specific probability distribution of the hidden state, and that there is a dependency between the previous hidden state and the hidden state to be transitioned next. The hidden state is a state not directly observed and, for example, is a state of the step action, which is a walking state or a non-walking state.

The information processing apparatus 100 labels the state of each step action as "walking" or "non-walking", for example, based on the evaluation values calculated for the first to tenth relationships. Then, the information processing apparatus 100 calculates the likelihood of the HMM for a partial period of the target period corresponding to a step action labeled as "walking", and regards the calculated likelihood as the likelihood as the walking period.

In the example illustrated in FIG. 27, as the hidden states of the step action, not only the simple states of "walking" and "non-walking", but also states obtained by subdividing the walking state into three states, namely, "initial walking state", "intermediate walking state", and "terminal walking state" are assumed to more precisely capture the dynamics of walking. Additionally, the walking state is further subdivided by distinguishing these three states by the right leg and the left leg, in order to cope with a case where the way of walking of the patient is different between the left leg and the right leg. Note that there may be a plurality of intermediate walking states.

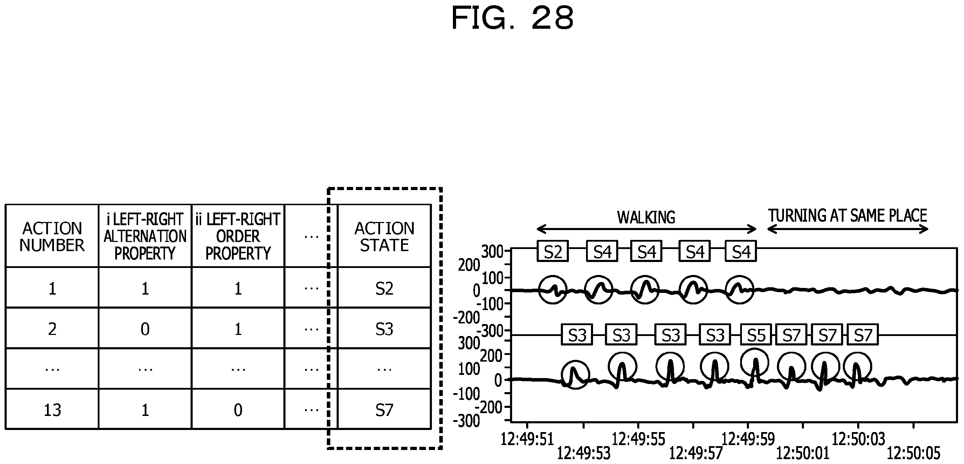

With this configuration, the information processing apparatus 100 is allowed to distinguish the walking state of "initial walking state (for example, the first step)" and "terminal walking state (for example, the final step)" having the tendency of moving by only a half step as compared to the walking state of "during walking", and is thus allowed to precisely find the boundary between "walking" and "non-walking". In addition, the information processing apparatus 100 distinguishes between the walking states of the left leg and the right leg so as to cope with a case where the patient has injured one leg, or the like. Next, description of FIG. 28 will be made.

In FIG. 28, the information processing apparatus 100 assigns a state having likeliness among the above-described seven states to an evaluation value series X calculated for the first to tenth relationships. The information processing apparatus 100 works out an action state series Y using, for example, the Viterbi algorithm expressed by following formula (14) such that a simultaneous probability p(X, Y) of the evaluation value series X and the action state series Y is maximized.

[Mathematical Formula 14]

$$p(X, Y) = \sum_{i=1}^{K} p(X_i | Y_i) p(y_i | y_{i-1}) \quad \dots (14)$$

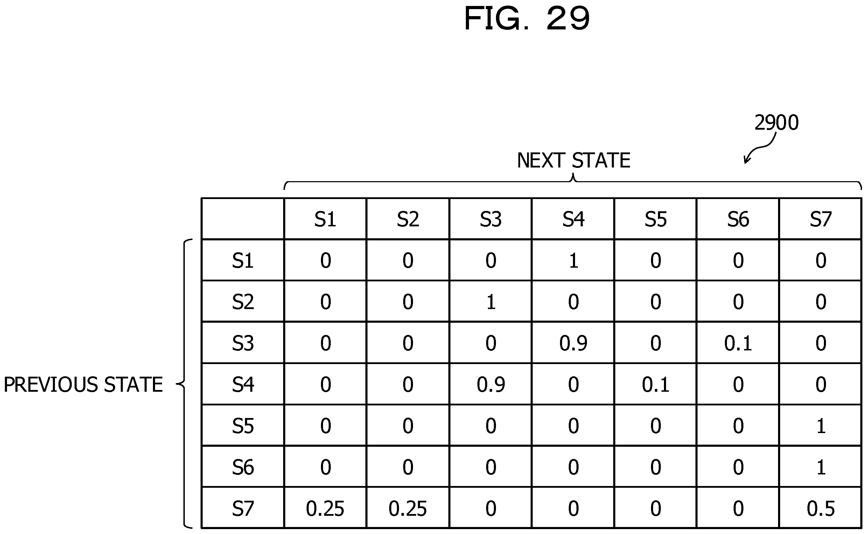

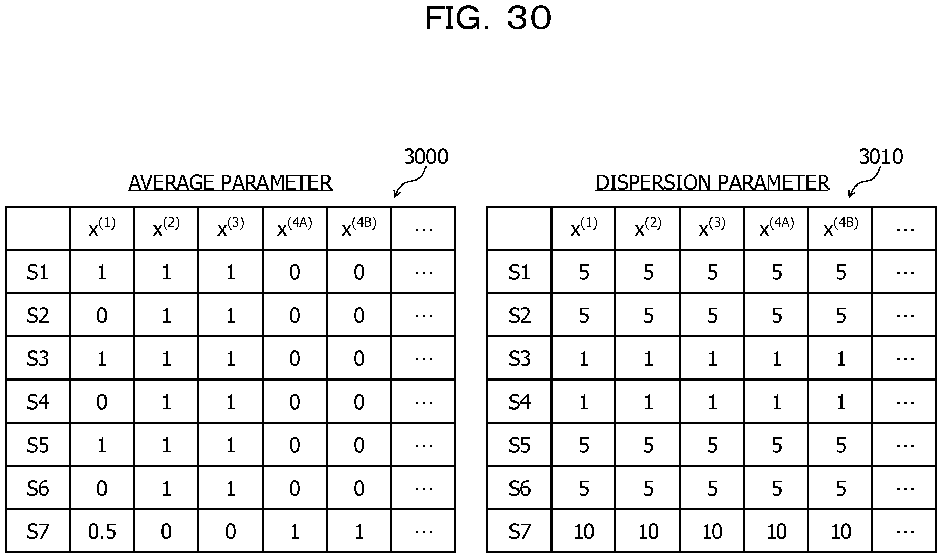

Here, a term $p(x_i|y_i)$ represents the observation probability. The observation probability will be described later with reference to, for example, FIG. 30. A term $p(y_i|y_{i-1})$ represents the state transition probability. The state transition probability will be described later with reference to, for example, FIG. 29. Next, description of FIG. 29 will be made.

As illustrated in FIG. 29, the state transition probability is expressed by table 2900. Here, the state has the property of transitioning alternately between the walking state of the right leg and the walking state of the left leg. For this reason, for the state transition probability $p(y_i|y_{i-1})$, the state transition probability is set such that the walking state related to the left leg and the walking state related to the right leg, which are obtained by subdividing the walking state, alternately occur. For example, if the previous state is S3, the state transition probability is set such that a state to be transitioned next is either S4 or S6.

In addition, the state transition probability is set such that the state transitions to the state S4 during walking after a state S1 of the first step at the start of walking, and the state transitions to the state S3 during walking after a state S2 of the first step at the start of walking. Furthermore, the state transition probability is set such that the state transitions to a state S7 of non-walking after the end of walking in a state S5 of the final step, and the state transitions to the state S7 of non-walking after the end of walking in the state S6 of the final step. Next, description of FIG. 30 will be made.

As illustrated in FIG. 30, the observation probability is expressed by tables 3000 and 3010. As for the observation probability $p(x_i|y_i)$, the probability distribution is heuristically set because the value taken by each evaluation value can be specified in each state of the walking state and the non-walking state. The shape of the probability distribution is designated using non-correlated multidimensional normal distribution by setting average parameters and dispersion parameters of the normal distribution as illustrated in tables 3000 and 3010.

For example, in the walking states S1, S2, S3, S4, S5, and S6, the average parameters of the evaluation value $x^{(2)}$ and the evaluation value $x^{(3)}$ are set to 1, and the average parameters of the evaluation value $x^{(4A)}$ and the evaluation value $x^{(4B)}$ are set to 0. Meanwhile, in the non-walking state s7, the average parameters of the evaluation value $x^{(2)}$ and the evaluation value $x^{(3)}$ are set to 0, and the average parameters of the evaluation value $x^{(4A)}$ and the evaluation value $x^{(4B)}$ are set to 1.

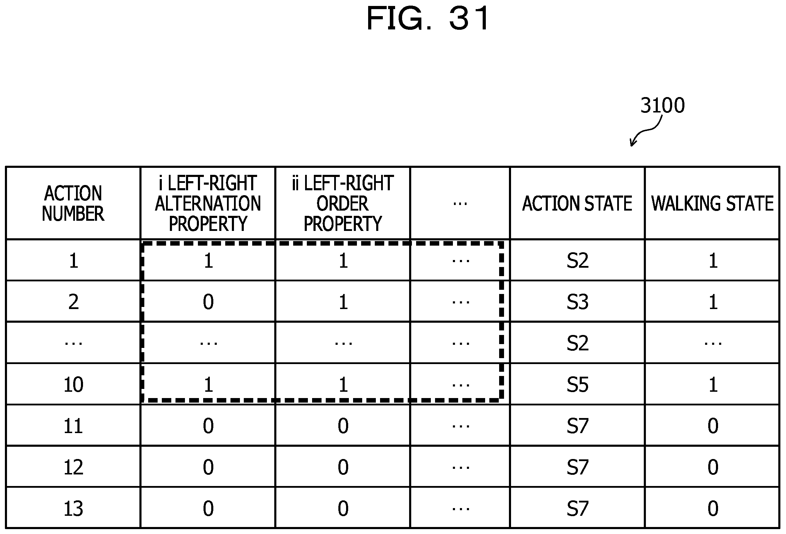

In addition, in the walking states S1, S3, and S5 corresponding to the step action of the right leg, the average parameter of the evaluation value $x^{(1)}$ is set to 1 and, in the walking states S2, S4, and S6 corresponding to the step action of the left leg, the average parameter of the evaluation value $x^{(1)}$ is set to 0. In the states S1 and S2 corresponding to the first steps at the start of walking and the states S5 and S6 corresponding to the final steps at the end of walking, since the angular velocity is prone to be smaller, each dispersion parameter is set large as compared to the states S3 and S4 during walking. Next, description of FIG. 31 will be made.

In FIG. 31, the information processing apparatus 100 specifies the action state series by applying the Viterbi algorithm using the state observation probability and the observation probability. For example, the information processing apparatus 100 specifies the action state series, and stores the specified action state series using an evaluation value management table 3100 by further associating the action state with the action number.

The information processing apparatus 100 specifies a period from the walking state of the first step to the walking state of the final step. If the specified period is a period corresponding to the number of steps greater than a predetermined number of steps, the information processing apparatus 100 specifies the specified period as a candidate period for the walking period. The predetermined number of steps is, for example, four steps.

In the example in FIG. 31, the information processing apparatus 100 specifies a period in which the action number 1 indicates the start time and the action number 10 indicates the end time. Since the number of steps in the specified period is ten steps, the information processing apparatus 100 specifies the specified period as a candidate period for the walking period. The information processing apparatus 100 sets the evaluation values for the specified candidate period into the evaluation value series X*.

Then, the information processing apparatus 100 calculates the likelihood of the simultaneous probability p(X*, Y*) calculated for the specified candidate period as the likelihood as the walking period, using following formula (15).
[Mathematical Formula 15]

$$\delta = p(X = X^{\star}, Y = Y^{\star}) = \sum_{i=Is}^{Ie} p(X_i|y_i)p(y_i|y_{i-1}) \quad \dots (15)$$

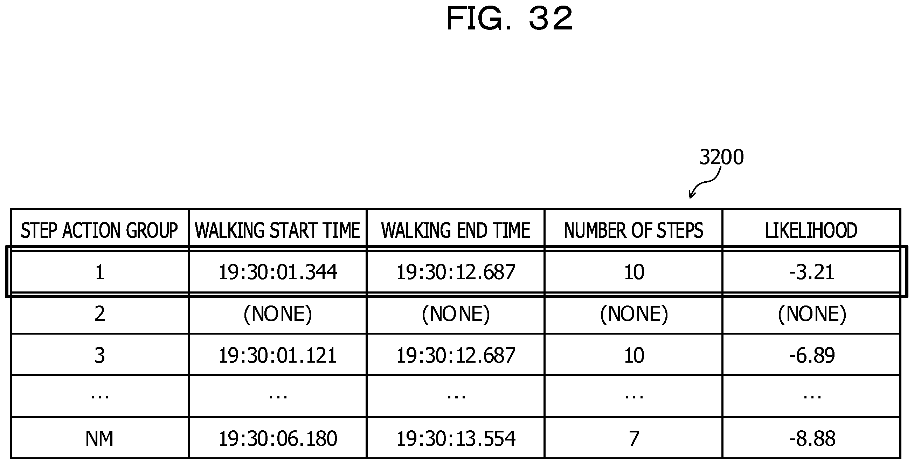

Here, according to formula (15), since the probability value increases as the number of steps decreases and the number of step actions decreases during the candidate period, the likelihood tends to increase. Therefore, the information processing apparatus 100 may further calculate, as the likelihood, an index value obtained by averaging the logarithmic value of above formula (15) with the number of steps K during the candidate period, using following formula (16). Next, description of FIG. 32 will be made.
[Mathematical Formula 16]

$$\delta = \frac{1}{K} log\, p(X = X^{\star}, Y = Y^{\star}) \quad \dots (16)$$

In FIG. 32, it is assumed that the information processing apparatus 100 has calculated the likelihood for each step action group. The information processing apparatus 100 stores the calculated likelihood using a likelihood management table 3200. The information processing apparatus 100 determines whether or not the maximum value of the likelihood exceeds a threshold.

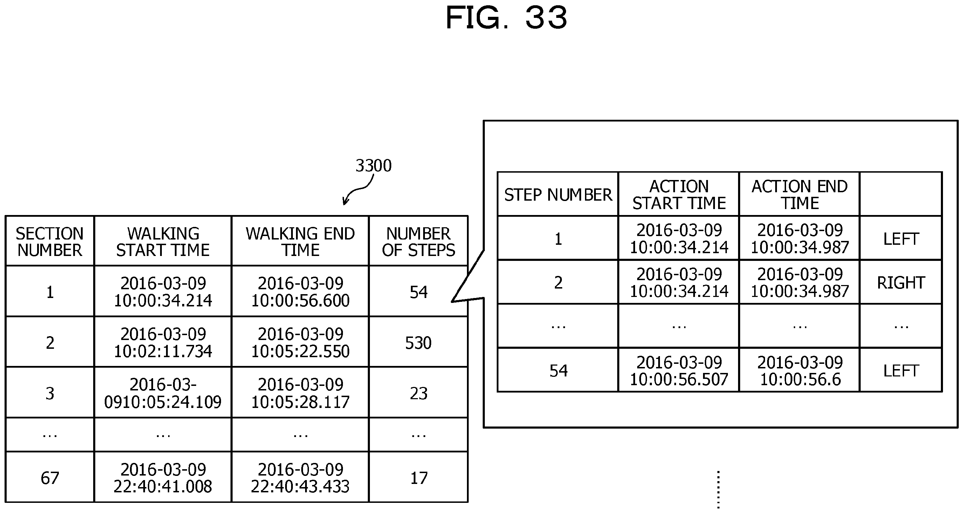

The information processing apparatus 100 selects, as a step action group having the likeliness as a step action group corresponding to the walking period of the patient, a step action group of which the likelihood has exceeded the threshold and reached the maximum value. Then, the information processing apparatus 100 specifies, for example, the start time and end time of the walking period of the patient. Next, description of FIG. 33 will be made.

In FIG. 33, it is assumed that the information processing apparatus 100 has similarly determined whether or not each target period is a walking period, and specified the start time and end time of the walking period. The information processing apparatus 100 stores the result of determination and the specified start time and end time of the walking period using a result management table 3300.

(Example in which Information Processing Apparatus 100 Outputs Predetermined Feature Quantity)

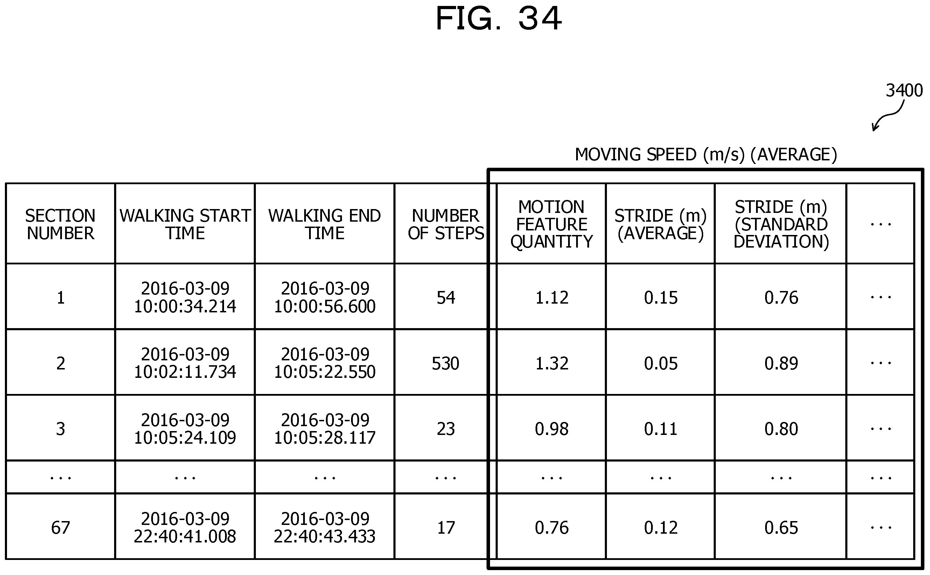

Next, an example in which the information processing apparatus 100 outputs a predetermined feature quantity will be described with reference to FIG. 34.

FIG. 34 is an explanatory diagram illustrating an example in which the information processing apparatus 100 outputs a predetermined feature quantity. In FIG. 34, the information processing apparatus 100 calculates a predetermined feature quantity in each walking period using the specified walking period and the start time and end time of the step action in the specified walking period. The predetermined feature quantity is, for example, the patient's stride, moving speed, moving time, walking cadence, impact when each leg lands, ground contact time of each leg, and the like. In addition, the predetermined feature quantity may be the start time and end time of each step action during the walking period. The information processing apparatus 100 further associates, for example, the calculated feature quantity with the step action to store using a result management table 3400 and the leg action management table 2400.

(Another Example of Operation of Applying Plurality of Band Pass Filters)

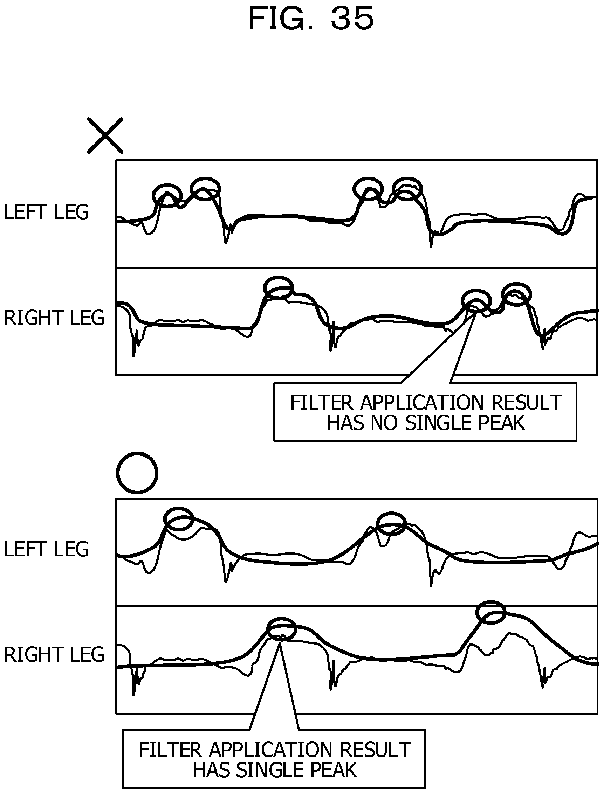

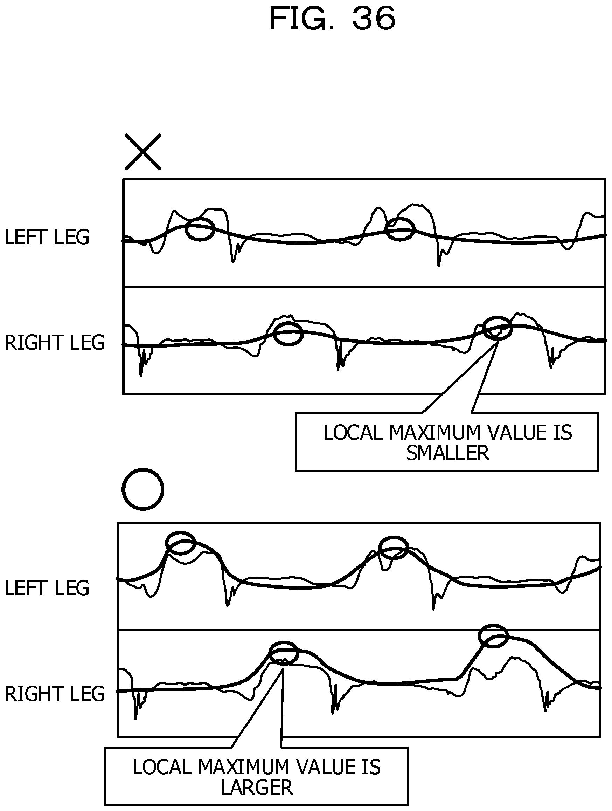

Next, another example of the operation of applying a plurality of band pass filters will be described with reference to FIGs. 35 and 36.

FIGs. 35 and 36 are explanatory diagrams illustrating another example of the operation of applying a plurality of band pass filters. As illustrated in FIGs. 35 and 36, the information processing apparatus 100 may not prepare a plurality of first parameters in advance when applying a plurality of band pass filters.

In FIG. 35, the information processing apparatus 100 sets a cutoff frequency such that, for example, the result of applying a certain band pass filter has a single peak property. The single peak property is a property that, after the angular velocity of one leg reached a local maximum value, the angular velocity of the one leg does not reach a local maximum value again before the angular velocity of the other leg reaches a local maximum value. The single peak property is, for example, a property that two or more step actions of one leg do not occur within a predetermined time after the step action of the same one leg. For example, if the single peak property does not appear as a result of applying a certain band pass filter, the information processing apparatus 100 applies a band pass filter having a wider frequency band than the frequency band of the applied band pass filter. Next, description of FIG. 36 will be made.

In FIG. 36, for example, the information processing apparatus 100 sets a cutoff frequency such that the local maximum value becomes equal to or more than a threshold as a result of applying a certain band pass filter. For example, if the local maximum value does not become equal to or more than the threshold as a result of applying a certain band pass filter, the information processing apparatus 100 applies a band pass filter having a wider frequency band than the frequency band of the applied band pass filter.

With this procedure, the information processing apparatus 100 does not have to use one of the results of applying each band pass filter of the plurality of band pass filters to the sensor data to determine whether or not the target period is a walking period. Therefore, the information processing apparatus 100 can suppress a rise in the amount of processing for determining whether or not the target period is a walking period.

(Another Example of Operation of Setting Plurality of Time Length Upper Limit Candidates for Time Length Upper Limit of Step Action)

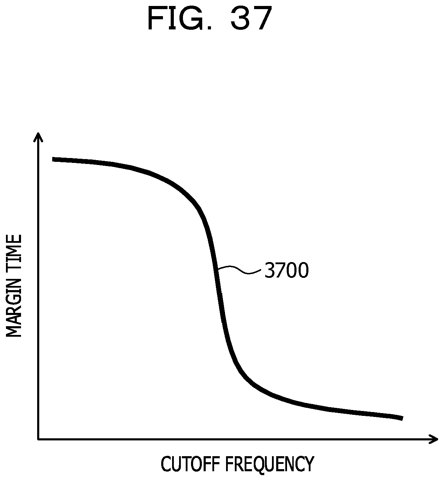

Next, another example of the operation of setting a plurality of time length upper limit candidates for the time length of the step action will be described with reference to FIG. 37.

FIG. 37 is an explanatory diagram illustrating another example of the operation of setting a plurality of time length upper limit candidates for the time length upper limit of the step action. As illustrated in FIG. 37, the information processing apparatus 100 may not prepare a plurality of second parameters in advance. The information processing apparatus 100 sets, for example, a second parameter to be a candidate for the time length upper limit of the step action according to the frequency band of the applied band pass filter. For example, the information processing apparatus 100 increases the second parameter as the frequency band is narrower, and decreases the second parameter as the frequency band is wider. The information processing apparatus 100 sets the second parameter smaller as the frequency band is wider, for example, by a function 3700.

With this configuration, the information processing apparatus 100 does not have to set a plurality of time length upper limit candidates for the time length upper limit of the step action, and can set a relatively preferable candidate for the time length upper limit of the step action. Therefore, the information processing apparatus 100 can suppress a rise in the amount of processing for determining whether or not the target period is a walking period.

(One Example of Overall Processing Procedure)

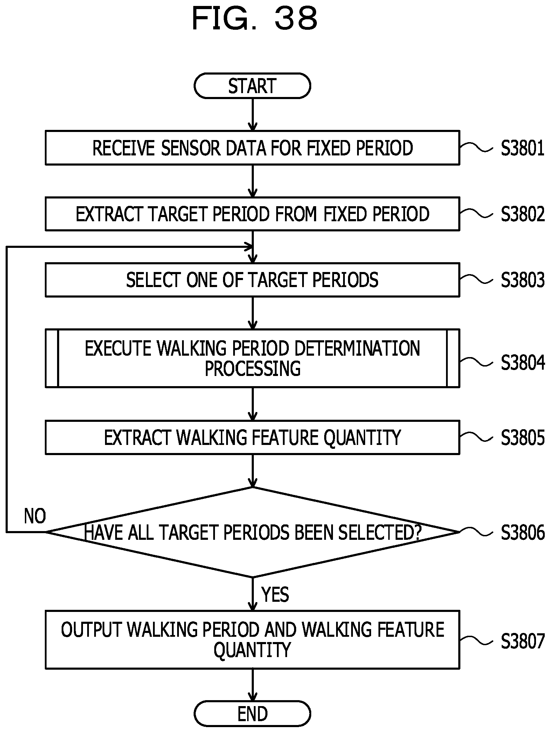

Next, an example of an overall processing procedure executed by the information processing apparatus 100 will be described with reference to FIG. 38.

FIG. 38 is a flowchart illustrating an example of an overall processing procedure. In FIG. 38, first, the information processing apparatus 100 receives sensor data for a fixed period from the measuring instrument 201 (step S3801). Next, the information processing apparatus 100 extracts a target period from the fixed period based on the sensor data (step S3802).

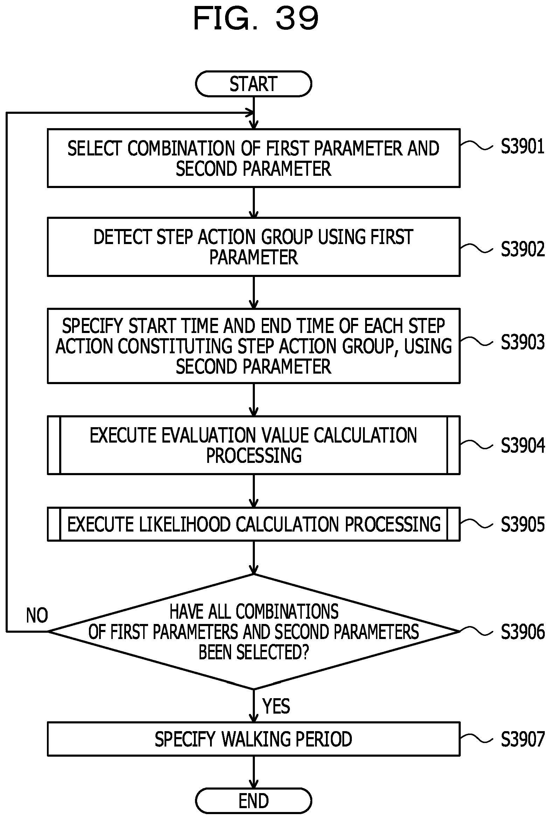

Then, the information processing apparatus 100 selects one of the target periods (step S3803). Next, the information processing apparatus 100 executes walking period determination processing described later with reference to FIG. 39 for the selected target period (step S3804). Then, the information processing apparatus 100 extracts a walking feature quantity (step S3805).

Next, the information processing apparatus 100 determines whether or not all of the extracted target periods have been selected (step S3806). Here, in a case where there is a target period that has not been selected (step S3806: No), the information processing apparatus 100 returns to the processing of step S3803.

On the other hand, in a case where all of the extracted target periods have been selected (step S3806: Yes), the information processing apparatus 100 outputs the walking period and the walking feature quantity (step S3807). Then, the information processing apparatus 100 ends the overall processing.

(Example of Walking Period Specifying Procedure)

Next, an example of a walking period specifying procedure executed by the information processing apparatus 100 will be described with reference to FIG. 39.

FIG. 39 is a flowchart illustrating an example of the walking period specifying procedure. In FIG. 39, first, the information processing apparatus 100 selects a combination of the first parameter and the second parameter based on the plurality of first parameters and the plurality of second parameters (step S3901).

Next, the information processing apparatus 100 applies a band pass filter to sensor data using the selected first parameter, and detects a step action group (step S3902). Then, the information processing apparatus 100 specifies the start time and end time of each step action constituting the step action group, using the selected second parameter (step S3903).

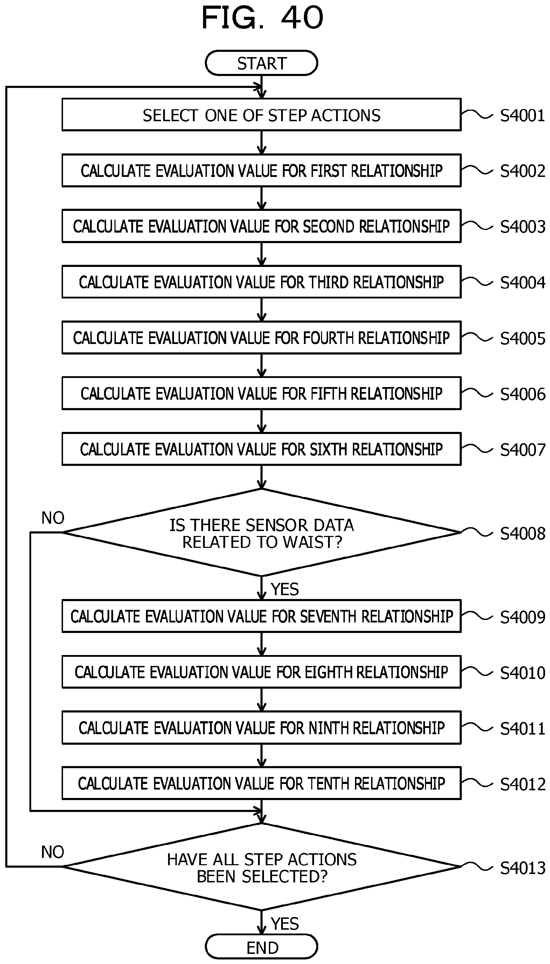

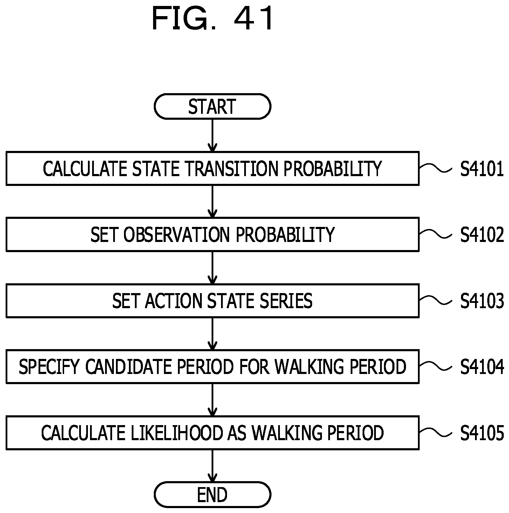

Next, the information processing apparatus 100 executes evaluation value calculation processing described later with reference to FIG. 40 (step S3904). Then, the information processing apparatus 100 executes likelihood calculation processing described later with reference to FIG. 41 (step S3905). Next, the information processing apparatus 100 determines whether or not all combinations of the first parameters and the second parameters have been selected (step S3906). Here, in a case where there is a combination that has not been selected (step S3906: No), the information processing apparatus 100 returns to the processing of step S3901.

On the other hand, in a case where all combinations have been selected (step S3906: Yes), the information processing apparatus 100 specifies a walking period, calculates a predetermined feature quantity in the walking period, and outputs the predetermined feature quantity in the walking period (step S3907). Then, the information processing apparatus 100 ends the walking period specifying processing.

(Example of Evaluation Value Calculation Processing Procedure)

Next, an example of an evaluation value calculation processing procedure executed by the information processing apparatus 100 will be described with reference to FIG. 40.

FIG. 40 is a flowchart illustrating an example of the evaluation value calculation processing procedure. In FIG. 40, first, the information processing apparatus 100 selects one of the step actions (step S4001).

Next, the information processing apparatus 100 calculates an evaluation value for the first relationship (step S4002). The information processing apparatus 100 also calculates an evaluation value for the second relationship (step S4003). The information processing apparatus 100 also calculates an evaluation value for the third relationship (step S4004). The information processing apparatus 100 also calculates an evaluation value for the fourth relationship (step S4005). The information processing apparatus 100 also calculates an evaluation value for the fifth relationship (step S4006). The information processing apparatus 100 also calculates an evaluation value for the sixth relationship (step S4007).

Then, the information processing apparatus 100 determines whether or not there is sensor data related to the waist (step S4008). Here, in a case where there is no sensor data (step S4008: No), the information processing apparatus 100 proceeds to the processing of step S4013.

On the other hand, in a case where there is sensor data (step S4008: Yes), the information processing apparatus 100 calculates an evaluation value for the seventh relationship (step S4009). The information processing apparatus 100 also calculates an evaluation value for the eighth relationship (step S4010). The information processing apparatus 100 also calculates an evaluation value for the ninth relationship (step S4011). The information processing apparatus 100 also calculates an evaluation value for the tenth relationship (step S4012).

Then, the information processing apparatus 100 determines whether or not all step actions have been selected (step S4013). Here, in a case where there is a step action that has not been selected (step S4013: No), the information processing apparatus 100 returns to the processing of step S4001.

On the other hand, in a case where all step actions have been selected (step S4013: Yes), the information processing apparatus 100 ends the evaluation value calculation processing.

(Example of Likelihood Calculation Processing Procedure)

Next, an example of a likelihood calculation processing procedure executed by the information processing apparatus 100 will be described with reference to FIG. 41.

FIG. 41 is a flowchart illustrating an example of the likelihood calculation processing procedure. In FIG. 41, first, the information processing apparatus 100 calculates a state transition probability (step S4101). Next, the information processing apparatus 100 sets an observation probability (step S4102). Then, the information processing apparatus 100 sets an action state series (step S4103).

Next, the information processing apparatus 100 specifies a candidate period for the walking period (step S4104). Then, the information processing apparatus 100 calculates the likelihood as the walking period for the candidate period for the walking period (step S4105). Thereafter, the information processing apparatus 100 ends the likelihood calculation processing.

As described above, according to the information processing apparatus 100, measurement information regarding the movement of each region of a plurality of regions of a patient can be acquired. According to the information processing apparatus 100, an action group that includes an action of each region in which a feature corresponding to a part of a walking motion action of the patient appears can be detected in a target period based on the measurement information. According to the information processing apparatus 100, in the detected action group, an evaluation value that indicates the degree of appearance of each relationship of a plurality of relationships produced between actions when a walking motion is performed by the patient can be calculated. According to the information processing apparatus 100, based on the evaluation value calculated for each relationship, whether or not the target period is a walking period in which a walking motion was performed by the patient can be determined. With this configuration, the information processing apparatus 100 can improve the accuracy of specifying the walking period.

According to the information processing apparatus 100, for a case where each band pass filter of a plurality of band pass filters is applied to measurement information, an action group that includes an action of each region in which a feature corresponding to a part of a walking motion action of the patient appears can be detected. According to the information processing apparatus 100, an evaluation value that indicates the degree of appearance of each relationship can be calculated for each of the detected action groups. With this configuration, the information processing apparatus 100 can improve the accuracy of specifying the walking period even in a case where the cycle of step action of each leg of the patient is different depending on the place where the patient walks, the surrounding environment when the patient walks, the condition of the patient, and the like, or different for each patient.

According to the information processing apparatus 100, in the action group, an evaluation value that indicates the degree of appearance of each relationship can be calculated for a case where the time length upper limit of each action is set to each candidate of a plurality of time length upper limit candidates. With this configuration, the information processing apparatus 100 can improve the accuracy of specifying the walking period even in a case where the time length of step action of each leg of the patient is different depending on the place where the patient walks, the surrounding environment when the patient walks, the condition of the patient, and the like, or different for each patient.

According to the information processing apparatus 100, a predetermined feature quantity in a target period determined to be a walking period can be output. With this configuration, the information processing apparatus 100 can notify the user of a predetermined feature quantity in order to allow the user to grasp the condition of the patient.

According to the information processing apparatus 100, an action period in which the patient is not in a stationary state can be specified based on the acquired measurement information, and set as a target period. With this configuration, the information processing apparatus 100 can reduce the number of target periods to be determined as to whether or not the target period is a walking period, and can reduce the amount of processing.

According to the information processing apparatus 100, a candidate period for the walking period can be specified from the target period based on the evaluation value calculated for each relationship, and the likelihood as a walking period according to the length of the specified candidate period can be calculated. According to the information processing apparatus 100, whether or not the candidate period is a walking period can be determined based on the calculated likelihood. With this configuration, the information processing apparatus 100 can improve the accuracy of specifying the walking period by further dividing the target period into the walking period and the non-walking period.

According to the information processing apparatus 100, if the evaluation value is a binary truth value that indicates whether or not the relationship has appeared, whether or not the target period is a walking period can be determined based on the result of logical operation using the evaluation value. With this configuration, the information processing apparatus 100 can reduce the amount of processing.

According to the information processing apparatus 100, the first to sixth relationships related to the action of the leg of the patient can be used as the plurality of relationships. With this configuration, the information processing apparatus 100 can evaluate the likeliness to the walking motion from a plurality of viewpoints regarding the action of the leg of the patient, and improve the accuracy of specifying the walking period.

According to the information processing apparatus 100, the seventh to tenth relationships related to the action of the leg of the patient and the body trunk of the patient can be used. With this configuration, the information processing apparatus 100 can evaluate the likeliness to the walking motion from a plurality of viewpoints regarding the actions of the leg of the patient and the body trunk of the patient, and improve the accuracy of specifying the walking period.

Note that the information processing method described in the present embodiment can be implemented by executing a prepared program on a computer such as a personal computer or a workstation. The information processing program described in the present embodiment is recorded on a computer-readable recording medium such as a hard disk, flexible disk, compact disk read only memory (CD-ROM), magneto-optical disk (MO), or digital versatile disc (DVD), and is read from the recording medium to be executed by the computer. In addition, the information processing program described in the present embodiment may be distributed via a network such as the Internet.

REFERENCE SIGNS LIST

100 information processing apparatus
101, 102, 1801, 1802 sensor data
200 information processing system
201 measuring instrument
210 network
300, 400 bus
301, 401 CPU
302, 402 memory
303, 403 network I/F
304 recording medium I/F
305 recording medium
404 sensor unit
405 timer unit
500 storage unit
501 acquisition unit
502 detection unit
503 evaluation unit
504 determination unit
505 calculation unit
506 output unit
711 to 714, 721 to 724, 811, 812, 821, 1301, 1302, 1401, 1402, 1501, 1601, 1602 step action
901 to 903, 1201, 1202 angular velocity
1001 to 1004, 1111 to 1114, 1121 to 1124 peak amplitude
1311 to 1313 vertical action
1411, 1412 longitudinal action
1511, 1611, 1612 body trunk advancing action
1700, 3300, 3400 result management table
2400 leg action management table

2500, 3100 evaluation value management table
2900, 3000, 3010 table
3200 likelihood management table
3700 function

**Claims**

1. An information processing apparatus comprising a control unit that:

   acquires measurement information regarding a movement of each region of a plurality of regions of a measurement target;
   based on the acquired measurement information, detects an action group that includes an action of the each region in which a feature corresponding to a part of a walking motion action of the measurement target appears in a target period;
   in the detected action group, calculates an evaluation value that indicates a degree of appearance of each relationship of a plurality of relationships produced between actions when a walking motion is performed by the measurement target; and
   based on the evaluation value calculated for the each relationship, determines whether or not the target period is a walking period in which the walking motion was performed by the measurement target.

2. The information processing apparatus according to claim 1, wherein
   the control unit:

   for a case where each band pass filter of a plurality of band pass filters is applied to the measurement information, detects action groups, each of which includes the action of the each region in which the feature corresponding to the part of the walking motion action of the measurement target appears; and
   calculates the evaluation value that indicates the degree of appearance of the each relationship for each of the detected action groups.

3. The information processing apparatus according to claim 1 or 2, wherein
   the control unit:

   for a case where a predetermined band pass filter is applied to the measurement information, sets a pass band of the predetermined band pass filter such that a result of applying the predetermined band pass filter has a single peak property and a peak value is equal to or more than a threshold; and
   for a case where the predetermined band notification filter is applied to the measurement information, detects the action group that includes the action of the each region in which the feature corresponding to the part of the walking motion action of the measurement target appears.

4. The information processing apparatus according to any one of claims 1 to 3, wherein
   the control unit:

   for a case where a local maximum value is detected using each threshold of a plurality of thresholds in the measurement information or a result of applying a predetermined band pass filter to the measurement information, detects action groups, each of which includes the action of the each region in which the feature corresponding to the part of the walking motion action of the measurement target appears; and
   calculates the evaluation value that indicates the degree of appearance of the each relationship for each of the detected action groups.

5. The information processing apparatus according to claim 1 or 2, wherein
   the control unit:
   in the action group, for a case where a time length upper limit of each action is set to each candidate of a plurality of time length upper limit candidates, detects time points that represent an action start and an action end of each action from the measurement information; and calculates the evaluation value that indicates the degree of appearance of the each relationship based on a result of the detection.

6. The information processing apparatus according to claim 3, wherein

the control unit:

in the action group, makes setting such that, as the set pass band is lowered, a time length upper limit of each action increases; detects time points that represent an action start and an action end of each action from the measurement information; and calculates the evaluation value that indicates the degree of appearance of the each relationship based on a result of the detection.

7. The information processing apparatus according to any one of claims 1 to 3, wherein
the control unit
outputs a predetermined feature quantity in the target period determined to be the walking period.

8. The information processing apparatus according to any one of claims 1 to 4, wherein
the control unit:
specifies an action period in which the measurement target is not in a stationary state, based on the acquired measurement information; and sets the specified action period as the target period.

9. The information processing apparatus according to any one of claims 1 to 5, wherein
the control unit:

based on the evaluation value calculated for the each relationship, specifies a candidate period for the walking period from the target period, and calculates a likelihood as the walking period according to a length of the specified candidate period; and
determines whether or not the candidate period is the walking period, based on the calculated likelihood.

10. The information processing apparatus according to claim 9, wherein
the control unit:

in the target period, assigns a walking state or a non-walking state to each action in the action group, using the evaluation value calculated for the each relationship; and
specifies the candidate period for the walking period from the target period, based on a result of assigning the walking state or the non-walking state.

11. The information processing apparatus according to claim 10, wherein

the control unit
assigns, to the action, one of at least two states including an initial state that represents a start of walking and a terminal state that represents an end of walking, as the walking state.

12. The information processing apparatus according to claim 10 or 11, wherein

the control unit
assigns, to the action, one of at least two states including a walking state related to a left leg and a walking state related to a right leg, as the walking state.

13. The information processing apparatus according to any one of claims 10 to 12, wherein

the control unit
assigns the walking state or the non-walking state to the each action, based on an inter-state transition probability defined between a current state and a state to be transitioned next in the walking state and the non-walking state.

14. The information processing apparatus according to any one of claims 1 to 6, wherein

the evaluation value is a binary truth value that indicates whether or not the each relationship has appeared, and the control unit
determines whether or not the target period is the walking period, based on a result of logical operation using the evaluation value.

15. The information processing apparatus according to any one of claims 1 to 7, wherein

the action includes an action of a leg of the measurement target, and
the plurality of relationships includes:

a first relationship in which actions of respective legs of the measurement target occur alternately;
a second relationship in which an action of one leg of the measurement target starts within a predetermined time after an action of the other leg of the measurement target ends;
a third relationship in which, during an action of one leg of the measurement target, an action amount of an action of the other leg of the measurement target falls within a predetermined range;
a fourth relationship in which feature quantities of a plurality of actions of one leg of the measurement target resemble each other;
a fifth relationship in which feature quantities of actions of respective legs of the measurement target resemble each other; or
a sixth relationship in which a rotation directions of actions of respective legs of the measurement target are identical to each other; or any combination thereof.

**16.** The information processing apparatus according to any one of 1 to 4, wherein

the action includes actions of a leg of the measurement target and a body trunk of the measurement target, and
the plurality of relationships includes:

a seventh relationship in which an action of the body trunk of the measurement target in a vertical direction occurs in a range of predetermined time before and after an action of each leg of the measurement target ends;
an eighth relationship in which, during an action of each leg of the measurement target, an action of the body trunk of the measurement target in an advance direction occurs;
a ninth relationship in which a rotation direction of an action of each leg of the measurement target is identical to a rotation direction of an action of the body trunk of the measurement target after the action of each leg of the measurement target ends; or
a tenth relationship in which a movement amount indicated by an action of each leg of the measurement target resembles a movement amount indicated by an action of the body trunk of the measurement target; or any combination thereof.

**17.** The information processing apparatus according to any one of claims 1 to 16, wherein the part of the walking motion action is one step of a walking motion.

**18.** An information processing system comprising a measuring instrument, and an information processing apparatus capable of communicating with the measuring instrument, wherein

the measuring instrument
generates measurement information regarding a movement of each region of a plurality of regions of a measurement target, and
the information processing apparatus:

acquires the measurement information from the measuring instrument;
based on the acquired measurement information, detects an action group that includes an action of the each region in which a feature corresponding to a part of a walking motion action of the measurement target appears in a target period;
in the detected action group, calculates an evaluation value that indicates a degree of appearance of each relationship of a plurality of relationships produced between actions when a walking motion is performed by the measurement target; and
based on the evaluation value calculated for the each relationship, determines whether or not the target period is a walking period in which the walking motion was performed by the measurement target.

**19.** An information processing method comprising:

acquiring, by a computer, measurement information regarding a movement of each region of a plurality of regions of a measurement target;
detecting, by the computer, based on the acquired measurement information, an action group that includes an action of the each region in which a feature corresponding to a part of a walking motion action of the measurement target appears in a target period;

calculating, by the computer, in the detected action group, an evaluation value that indicates a degree of appearance of each relationship of a plurality of relationships produced between actions when a walking motion is performed by the measurement target; and

determining, by the computer, based on the evaluation value calculated for the each relationship, whether or not the target period is a walking period in which the walking motion was performed by the measurement target.

# FIG. 1

(1-1) ACQUIRE MEASUREMENT INFORMATION

SENSOR DATA ⟶ 101

SENSOR DATA ⟶ 102

(1-2) DETECT ACTION GROUP

○ : ACTION POINT

(1-3) EVALUATE RELATIONSHIP BETWEEN ACTIONS

DO LEFT LEG AND RIGHT LEG PERFORM STEP ACTION ALTERNATELY?

DO FEATURE QUANTITIES OF INDIVIDUAL STEPS OF ONE LEG RESEMBLE EACH OTHER?

DO LEFT LEG AND RIGHT LEG PERFORM STEP ACTION IN ORDER?

DOES ONE LEG SUPPORT WEIGHT OF PATIENT WHILE OTHER LEG IS PERFORMING STEP ACTION?

(1-4) DETERMINE WALKING PERIOD

# FIG. 2

# FIG. 3

# FIG. 4

401
CPU

402
MEMORY

201

400

403
NETWORK I/F

404
SENSOR UNIT

405
TIMER UNIT

210
NETWORK

# FIG. 5

EP 3 626 170 A1

# FIG. 6

# FIG. 8

ii LEFT-RIGHT ORDER PROPERTY

LEFT LEG

811

812

RIGHT LEG

801

802

821

TIME

EP 3 626 170 A1

# FIG. 9

iii LEFT-RIGHT SUPPORTABILITY

# FIG. 10

iv ONE-SIDE RESEMBLANCE

ACTION FEATURES ARE SUBSTANTIALLY SAME AS EACH OTHER

LEFT LEG

RIGHT LEG

10:51:12    10:51:13    10:51:14    10:51:15    10:51:16

# FIG. 11

RATIO OF ACTION FEATURES BETWEEN LEFT AND RIGHT ARE SUBSTANTIALLY SAME AS EACH OTHER

1111

v BOTH-SIDE RESEMBLANCE

LEFT LEG

RIGHT LEG

EP 3 626 170 A1

# FIG. 12

vi LEFT-RIGHT ROTATION PROPERTY

ROTATION ANGLE IN LATERAL DIRECTION

1202

LEFT LEG

RIGHT LEG

1201

TIME

EP 3 626 170 A1

# FIG. 13

EP 3 626 170 A1

# FIG. 14

viii VERTICAL PARALLEL ADVANCE PROPERTY

ANGULAR VELOCITY OF RIGHT LEG

400
200
0
-200
-400

1401

1402

LONGITUDINAL ACCELERATION OF WAIST

16
14
12
10
8
6
4

14:30:46

1411

14:30:47

1412

14:30:48

ADVANCING ACTION

EP 3 626 170 A1

# FIG. 15

EP 3 626 170 A1

ix VERTICAL ROTATION
PROPERTY

ANGULAR VELOCITY
OF RIGHT LEG

400
200
0
-200
-400

1501

ANGULAR VELOCITY
OF WAIST

16
14
12
10
8
6
4

14:30:46          14:30:47          14:30:48

1511

# FIG. 16

EP 3 626 170 A1

FIG. 17

1700

| | CERTAINTY FACTOR OF RELATIONSHIP i | ... | CERTAINTY FACTOR OF RELATIONSHIP vi | COMPREHENSIVE EVALUATION VALUE | WALKING DETERMINATION RESULT |
|---|---|---|---|---|---|
| ACTION GROUP 1 | 0.99 | ... | 0.45 | 0.046 | |
| ACTION GROUP 2 | 0.99 | ... | 0.98 | 0.121 | ○ |
| ... | ... | ... | ... | ... | |
| ACTION GROUP N | 0.01 | ... | 0.5 | 0.01 | |

EP 3 626 170 A1

# FIG. 18

[MOUNTING EXAMPLE]

201

RIGHT LEG

201

LEFT LEG

201    MOTION SENSOR

LEFT LEG

RIGHT LEG

1801

1802

TIME

EP 3 626 170 A1

FIG. 19

SAGITTAL PLANE

TRAVERSE PLANE

CORONAL PLANE

# FIG. 20

# FIG. 21

WINDOW

1  2    3        4      5          6   7  ← TARGET PERIOD

TARGET PERIOD 1

12:19:19  12:19:21  12:19:23  12:19:25  12:19:27  12:19:29  12:19:31  12:19:33  12:19:35

EP 3 626 170 A1

# FIG. 22A

[FIRST PARAMETER]

| NUMBER | CUTOFF FREQUENCY |
|--------|------------------|
| 1 | 0.1 Hz |
| 2 | 0.5 Hz |
| ... | ... |
| N | 3.0 Hz |

LEFT LEG

RIGHT LEG

FREQUENCY BAND PASSED BY FILTER

DETECTOR

~0.1 Hz    FREQUENCY    ~0.5 Hz    ....    ~3 Hz

BAND PASS FILTER

WAVEFORM AFTER FILTER IS APPLIED

....

DETECT STEP ACTION

STEP ACTION GROUP

....

EP 3 626 170 A1

# FIG. 22B

LEFT LEG

RIGHT LEG

[THIRD PARAMETER]

| NUMBER | THRESHOLD |
|--------|-----------|
| 1 | 20 |
| 2 | 40 |
| ... | ... |
| L | 200 |

THRESHOLD

.......

DETECT STEP ACTION

LEFT LEG

RIGHT LEG

LEFT LEG

RIGHT LEG

EP 3 626 170 A1

# FIG. 23

EP 3 626 170 A1

LEFT LEG

RIGHT LEG

MARGIN TIME

[SECOND PARAMETER]

| NUMBER | MARGIN TIME |
|--------|-------------|
| 1 | 0.3 SECONDS |
| 2 | 0.5 SECONDS |
| ... | ... |
| M | 2.0 SECONDS |

SPECIFY START TIME AND END TIME

LEFT LEG

RIGHT LEG

LEFT LEG

RIGHT LEG

# FIG. 24

TARGET PERIOD

DETECT STEP ACTION

SPECIFY START TIME AND END TIME OF STEP ACTION

N × M STEP ACTION GROUPS

2400

| ACTION NUMBER | ACTION POINT | START TIME | END TIME | LEFT SIDE OR RIGHT SIDE |
|---|---|---|---|---|
| 1 | 19:30:01.344 | 19:30:01.023 | 19:30:01.609 | 左 |
| 2 | 19:30:02.785 | 19:30:02.461 | 19:30:03.021 | 右 |
| … | … | … | … | … |
| 27 | 19:30:01.344 | 19:30:01.023 | 19:30:01.609 | 右 |

EP 3 626 170 A1

FIG. 25

2500

| ACTION NUMBER | i LEFT-RIGHT ALTERNATION PROPERTY | ii LEFT-RIGHT ORDER PROPERTY | iii LEFT-RIGHT SUPPORTABILITY | ... |
|---|---|---|---|---|
| 1 | 1 | 1 | 1 | ... |
| 2 | 0 | 1 | 1 | ... |
| ... | ... | ... | ... | ... |
| 13 | 1 | 0 | 0 | ... |

TEN TYPES OF EVALUATION VALUES

# FIG. 26

EP 3 626 170 A1

[EVALUATION VALUE]

| ACTION NUMBER | ... | ii LEFT-RIGHT ORDER PROPERTY | iii LEFT-RIGHT ONE-SIDE RESEMBLANCE | ... |
|---|---|---|---|---|
| 1 | ... | 1 | 0.1 | ... |
| 2 | ... | 1 | 0.2 | ... |
| ... | ... | ... | ... | ... |
| 13 | ... | 0 | 4.7 | ... |

[EVALUATION VALUE]

| ACTION NUMBER | ... | ii LEFT-RIGHT ORDER PROPERTY | iii LEFT-RIGHT ONE-SIDE RESEMBLANCE | ... |
|---|---|---|---|---|
| 1 | ... | 1 | 1 | ... |
| 2 | ... | 1 | 1 | ... |
| ... | ... | ... | ... | ... |
| 13 | ... | 0 | 0 | ... |

VALUE OBTAINED BY ADDING ALL OF LEFT INDIVIDUAL EVALUATION VALUES IS USED AS FINAL EVALUATION VALUE

# FIG. 27

RIGHT LEG     S1     S3     S5

S7

LEFT LEG     S2     S4     S6

INITIAL WALKING STATE     INTERMEDIATE WALKING STATE     TERMINAL WALKING STATE     NON-WALKING STATE

STATE REPRESENTING WALKING     STATE REPRESENTING NON-WALKING

EP 3 626 170 A1

# FIG. 28

EP 3 626 170 A1

| ACTION NUMBER | i LEFT-RIGHT ALTERNATION PROPERTY | ii LEFT-RIGHT ORDER PROPERTY | ... | ACTION STATE |
|---|---|---|---|---|
| 1 | 1 | 1 | ... | S2 |
| 2 | 0 | 1 | ... | S3 |
| ... | ... | ... | ... | ... |
| 13 | 1 | 0 | ... | S7 |

# FIG. 29

EP 3 626 170 A1

NEXT STATE — 2900

| PREVIOUS STATE | S1 | S2 | S3 | S4 | S5 | S6 | S7 |
|---|---|---|---|---|---|---|---|
| S1 | 0 | 0 | 0 | 1 | 0 | 0 | 0 |
| S2 | 0 | 0 | 1 | 0 | 0 | 0 | 0 |
| S3 | 0 | 0 | 0 | 0.9 | 0 | 0.1 | 0 |
| S4 | 0 | 0 | 0.9 | 0 | 0.1 | 0 | 0 |
| S5 | 0 | 0 | 0 | 0 | 0 | 0 | 1 |
| S6 | 0 | 0 | 0 | 0 | 0 | 0 | 1 |
| S7 | 0.25 | 0.25 | 0 | 0 | 0 | 0 | 0.5 |

# FIG. 30

AVERAGE PARAMETER 3000

| | $x^{(1)}$ | $x^{(2)}$ | $x^{(3)}$ | $x^{(4A)}$ | $x^{(4B)}$ | ... |
|---|---|---|---|---|---|---|
| S1 | 1 | 1 | 1 | 0 | 0 | ... |
| S2 | 0 | 1 | 1 | 0 | 0 | ... |
| S3 | 1 | 1 | 1 | 0 | 0 | ... |
| S4 | 0 | 1 | 1 | 0 | 0 | ... |
| S5 | 1 | 1 | 1 | 0 | 0 | ... |
| S6 | 0 | 1 | 1 | 0 | 0 | ... |
| S7 | 0.5 | 0 | 0 | 1 | 1 | ... |

DISPERSION PARAMETER 3010

| | $x^{(1)}$ | $x^{(2)}$ | $x^{(3)}$ | $x^{(4A)}$ | $x^{(4B)}$ | ... |
|---|---|---|---|---|---|---|
| S1 | 5 | 5 | 5 | 5 | 5 | ... |
| S2 | 5 | 5 | 5 | 5 | 5 | ... |
| S3 | 1 | 1 | 1 | 1 | 1 | ... |
| S4 | 1 | 1 | 1 | 1 | 1 | ... |
| S5 | 5 | 5 | 5 | 5 | 5 | ... |
| S6 | 5 | 5 | 5 | 5 | 5 | ... |
| S7 | 10 | 10 | 10 | 10 | 10 | ... |

EP 3 626 170 A1

# FIG. 31

3100

| ACTION NUMBER | i LEFT-RIGHT ALTERNATION PROPERTY | ii LEFT-RIGHT ORDER PROPERTY | ... | ACTION STATE | WALKING STATE |
|---|---|---|---|---|---|
| 1 | 1 | 1 | ... | S2 | 1 |
| 2 | 0 | 1 | ... | S3 | 1 |
| ... | ... | ... | ... | S2 | ... |
| 10 | 1 | 1 | ... | S5 | 1 |
| 11 | 0 | 0 | ... | S7 | 0 |
| 12 | 0 | 0 | ... | S7 | 0 |
| 13 | 0 | 0 | ... | S7 | 0 |

EP 3 626 170 A1

# FIG. 32

3200

| STEP ACTION GROUP | WALKING START TIME | WALKING END TIME | NUMBER OF STEPS | LIKELIHOOD |
|---|---|---|---|---|
| 1 | 19:30:01.344 | 19:30:12.687 | 10 | -3.21 |
| 2 | (NONE) | (NONE) | (NONE) | (NONE) |
| 3 | 19:30:01.121 | 19:30:12.687 | 10 | -6.89 |
| … | … | … | … | … |
| NM | 19:30:06.180 | 19:30:13.554 | 7 | -8.88 |

# FIG. 33

3300

| SECTION NUMBER | WALKING START TIME | WALKING END TIME | NUMBER OF STEPS |
|---|---|---|---|
| 1 | 2016-03-09 10:00:34.214 | 2016-03-09 10:00:56.600 | 54 |
| 2 | 2016-03-09 10:02:11.734 | 2016-03-09 10:05:22.550 | 530 |
| 3 | 2016-03-0910:05:24.109 | 2016-03-09 10:05:28.117 | 23 |
| ... | ... | ... | ... |
| 67 | 2016-03-09 22:40:41.008 | 2016-03-09 22:40:43.433 | 17 |

| STEP NUMBER | ACTION START TIME | ACTION END TIME | |
|---|---|---|---|
| 1 | 2016-03-09 10:00:34.214 | 2016-03-09 10:00:34.987 | LEFT |
| 2 | 2016-03-09 10:00:34.214 | 2016-03-09 10:00:34.987 | RIGHT |
| ... | ... | ... | ... |
| 54 | 2016-03-09 10:00:56.507 | 2016-03-09 10:00:56.6 | LEFT |

EP 3 626 170 A1

# FIG. 34

3400

EP 3 626 170 A1

MOVING SPEED (m/s) (AVERAGE)

| SECTION NUMBER | WALKING START TIME | WALKING END TIME | NUMBER OF STEPS | MOTION FEATURE QUANTITY | STRIDE (m) (AVERAGE) | STRIDE (m) (STANDARD DEVIATION) | · · · |
|---|---|---|---|---|---|---|---|
| 1 | 2016-03-09 10:00:34.214 | 2016-03-09 10:00:56.600 | 54 | 1.12 | 0.15 | 0.76 | · · · |
| 2 | 2016-03-09 10:02:11.734 | 2016-03-09 10:05:22.550 | 530 | 1.32 | 0.05 | 0.89 | · · · |
| 3 | 2016-03-09 10:05:24.109 | 2016-03-09 10:05:28.117 | 23 | 0.98 | 0.11 | 0.80 | · · · |
| · · · | · · · | · · · | · · · | · · · | · · · | · · · | · · · |
| 67 | 2016-03-09 22:40:41.008 | 2016-03-09 22:40:43.433 | 17 | 0.76 | 0.12 | 0.65 | · · · |

# FIG. 35

LEFT LEG

RIGHT LEG

FILTER APPLICATION RESULT
HAS NO SINGLE PEAK

LEFT LEG

RIGHT LEG

FILTER APPLICATION RESULT
HAS SINGLE PEAK

# FIG. 36

LEFT LEG

RIGHT LEG

LOCAL MAXIMUM VALUE IS SMALLER

LEFT LEG

RIGHT LEG

LOCAL MAXIMUM VALUE IS LARGER

# FIG. 37

# FIG. 38

```
START
  │
  ▼
RECEIVE SENSOR DATA FOR FIXED PERIOD          ⌇ S3801
  │
  ▼
EXTRACT TARGET PERIOD FROM FIXED PERIOD       ⌇ S3802
  │
  ▼
SELECT ONE OF TARGET PERIODS                  ⌇ S3803
  │
  ▼
EXECUTE WALKING PERIOD DETERMINATION
PROCESSING                                    ⌇ S3804
  │
  ▼
EXTRACT WALKING FEATURE QUANTITY              ⌇ S3805
  │
  ▼
HAVE ALL TARGET PERIODS BEEN SELECTED?        ⌇ S3806
  │ YES
  ▼
OUTPUT WALKING PERIOD AND WALKING FEATURE
QUANTITY                                       ⌇ S3807
  │
  ▼
END
```

NO

# FIG. 39

START

SELECT COMBINATION OF FIRST PARAMETER AND SECOND PARAMETER — S3901

DETECT STEP ACTION GROUP USING FIRST PARAMETER — S3902

SPECIFY START TIME AND END TIME OF EACH STEP ACTION CONSTITUTING STEP ACTION GROUP, USING SECOND PARAMETER — S3903

EXECUTE EVALUATION VALUE CALCULATION PROCESSING — S3904

EXECUTE LIKELIHOOD CALCULATION PROCESSING — S3905

HAVE ALL COMBINATIONS OF FIRST PARAMETERS AND SECOND PARAMETERS BEEN SELECTED? — S3906

NO

YES

SPECIFY WALKING PERIOD — S3907

END

# FIG. 40

START

SELECT ONE OF STEP ACTIONS — S4001

CALCULATE EVALUATION VALUE FOR FIRST RELATIONSHIP — S4002

CALCULATE EVALUATION VALUE FOR SECOND RELATIONSHIP — S4003

CALCULATE EVALUATION VALUE FOR THIRD RELATIONSHIP — S4004

CALCULATE EVALUATION VALUE FOR FOURTH RELATIONSHIP — S4005

CALCULATE EVALUATION VALUE FOR FIFTH RELATIONSHIP — S4006

CALCULATE EVALUATION VALUE FOR SIXTH RELATIONSHIP — S4007

IS THERE SENSOR DATA RELATED TO WAIST? — S4008

NO

YES

CALCULATE EVALUATION VALUE FOR SEVENTH RELATIONSHIP — S4009

CALCULATE EVALUATION VALUE FOR EIGHTH RELATIONSHIP — S4010

CALCULATE EVALUATION VALUE FOR NINTH RELATIONSHIP — S4011

CALCULATE EVALUATION VALUE FOR TENTH RELATIONSHIP — S4012

HAVE ALL STEP ACTIONS BEEN SELECTED? — S4013

NO

YES

END

# FIG. 41

```
        ┌─────────────┐
        │   START     │
        └──────┬──────┘
               ↓
┌──────────────────────────────────────┐
│ CALCULATE STATE TRANSITION PROBABILITY│ ～ S4101
└──────────────────┬───────────────────┘
                   ↓
┌──────────────────────────────────────┐
│      SET OBSERVATION PROBABILITY      │ ～ S4102
└──────────────────┬───────────────────┘
                   ↓
┌──────────────────────────────────────┐
│        SET ACTION STATE SERIES        │ ～ S4103
└──────────────────┬───────────────────┘
                   ↓
┌──────────────────────────────────────┐
│ SPECIFY CANDIDATE PERIOD FOR WALKING PERIOD │ ～ S4104
└──────────────────┬───────────────────┘
                   ↓
┌──────────────────────────────────────┐
│  CALCULATE LIKELIHOOD AS WALKING PERIOD │ ～ S4105
└──────────────────┬───────────────────┘
                   ↓
        ┌─────────────┐
        │    END      │
        └─────────────┘
```

## INTERNATIONAL SEARCH REPORT

| International application No. |
| --- |
| PCT/JP2017/018935 |

A. CLASSIFICATION OF SUBJECT MATTER
*A61B5/11*(2006.01)i, *A61H1/02*(2006.01)i

According to International Patent Classification (IPC) or to both national classification and IPC

B. FIELDS SEARCHED

Minimum documentation searched (classification system followed by classification symbols)
A61B5/11, A61H1/02

Documentation searched other than minimum documentation to the extent that such documents are included in the fields searched
Jitsuyo Shinan Koho          1922–1996   Jitsuyo Shinan Toroku Koho   1996–2017
Kokai Jitsuyo Shinan Koho    1971–2017   Toroku Jitsuyo Shinan Koho   1994–2017

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| A | US 2017/0095181 A1   (LUMO BODYTECH, INC.), 06 April 2017 (06.04.2017), entire text; all drawings (Family: none) | 1–19 |
| A | JP 2009-538636 A   (Koninklijke Philips Electronics N.V.), 12 November 2009 (12.11.2009), entire text; all drawings & US 2009/0204030 A1     & WO 2007/102134 A2 entire text; all drawings & EP 1993444 A1              & CN 101938940 A | 1–19 |
| A | WO 2017/065087 A1   (Alps Electric Co., Ltd.), 20 April 2017 (20.04.2017), entire text; all drawings (Family: none) | 1–19 |

| ☒ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 03 August 2017 (03.08.17) | 15 August 2017 (15.08.17) |

| Name and mailing address of the ISA/ Japan Patent Office 3-4-3,Kasumigaseki,Chiyoda-ku, Tokyo 100-8915,Japan | Authorized officer |
| | Telephone No. |

Form PCT/ISA/210 (second sheet) (January 2015)

**INTERNATIONAL SEARCH REPORT**

| International application No. |
| --- |
| PCT/JP2017/018935 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 2003-6608 A  (Seiko Instruments Inc.), 10 January 2003 (10.01.2003), entire text; all drawings (Family: none) | 1-19 |
| A | JP 2017-435 A  (Kao Corp.), 05 January 2017 (05.01.2017), entire text; all drawings (Family: none) | 1-19 |
| A | JP 2016-158887 A  (Fujitsu Ltd.), 05 September 2016 (05.09.2016), entire text; all drawings & US 2016/0256078 A1 entire text; all drawings & EP 3064133 A1 | 1-19 |
| A | JP 2011-177278 A  (Tokyo Institute of Technology), 15 September 2011 (15.09.2011), entire text; all drawings (Family: none) | 1-19 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016106948 A
- WO 2014091583 A
- JP 2014511189 A

**Non-patent literature cited in the description**

- **BAO.** *Activity Recognition from User-Annotated Acceleration Data,* 2004
- **FRACCALO.** *Real-world Gyroscope-based Gait Event Detection and Gait Feature Extraction,* 2014